# EUROPEAN PATENT APPLICATION

(11) **EP 4 752 134 A1**
(43) Date of publication of application: **03.06.2026**
(21) Application number: 24845153.6
(22) Date of filing: 16.05.2024
(51) Int. Cl.: C07C 261/02, C08G 61/00, C08G 73/06

(54) **CYANATE ESTER COMPOUND AND METHOD FOR PRODUCING SAME, RESIN COMPOSITION, AND CURED PRODUCT**

(30) Priority: 27.07.2023 JP 2023122171
(71) Applicant: MITSUBISHI GAS CHEMICAL COMPANY, INC., Chiyoda-ku Tokyo 100-8324 (JP)
(72) Inventor: KATAGIRI, Masayuki, Niigata-shi, Niigata 950-3112 (JP); IWAMOTO, Shinpei, Niigata-shi, Niigata 950-3112 (JP); YASUDA, Yoshihiro, Niigata-shi, Niigata 950-3112 (JP)
(74) Representative: Hoffmann Eitle
(86) International application number: PCT/JP2024/018151
(87) International publication number: WO 2025/022769

(57) **Abstract**

In the cyanate ester compound having two or more cyanate groups in a molecule according to the present invention, a content of a compound having a triazine ring is 4.0% by area or less on HPLC area percentage.

## Description

### Technical Field

The present invention relates to a cyanate ester compound and a method for producing the same, a resin composition, and a cured product.

### Background Art

Cyanate ester compounds are known as thermosetting resins that form a triazine ring by curing. Cured products obtained from the cyanate ester compounds have properties such as a high glass transition temperature, a low dielectric constant and dissipation factor, and excellent electrical insulation properties and flame retardancy. The cyanate ester compounds have heretofore been widely used as starting materials for various functional polymer materials such as composite materials for structures, adhesives, electrical insulating materials, and electrical and electronic components.

Hence, cyanate ester having better properties and a method for producing the same are under development (e.g., Patent Documents 1 and 2).

### Citation List

### Patent Document

Patent Document 1: Japanese Patent Laid-Open No. 2005-264154
Patent Document 2: WO2014/065422A1

### Summary of Invention

### Technical Problem

However, for cyanate ester compounds described in Patent Documents 1 and 2, it is difficult to control polymerization reaction at the time of cured product production and to obtain a cured product having desired characteristics.

The present invention has been made in light of such problems, and an object of the present invention is to provide a cyanate ester compound that facilitates controlling polymerization reaction at the time of cured product production and is suitably used in the production of a cured product having desired characteristics, and a method for producing the same, a resin composition, and a cured product.

### Solution to Problem

The present inventors have conducted diligent studies to attain the object described above and consequently completed the present invention by finding that the object can be attained by using a specific cyanate ester compound.

Specifically, the present invention is as follows.

[1] A cyanate ester compound having two or more cyanate groups in a molecule, wherein a content of a compound having a triazine ring is 4.0% by area or less on HPLC area percentage.
[2] The cyanate ester compound according to [1], wherein the cyanate ester compound is a compound represented by the following formula (1) or a compound represented by the following formula (2): 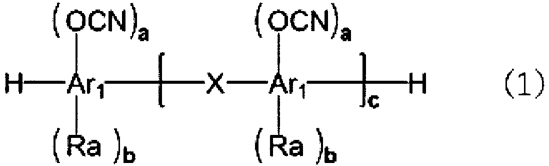
   wherein each Ar₁ independently represents an aromatic ring, each Ra independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms, a represents the number of cyanate groups bonded to Ar₁ and each independently represents an integer of 1 or larger and 3 or smaller, b represents the number of Ra bonded to Ar₁ and each independently represents a number determined by subtracting (a + 2) from the number of substitutable substituents in Ar₁, c is an integer of 1 or larger and 50 or smaller, and each X independently represents a single bond, a divalent organic group having 1 or more and 50 or less carbon atoms in which a hydrogen atom is optionally replaced with a heteroatom, a divalent organic group having 1 or more and 10 or less nitrogen atoms, a carbonyl group, a carboxy group, a carbonyl dioxide group, a sulfonyl group, a divalent sulfur atom, or a divalent oxygen atom, and 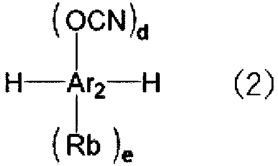
   wherein Ar₂ represents an aromatic ring, each Rb independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms, d represents the number of cyanate groups bonded to Ar₂ and is an integer of 2 or larger and 3 or smaller, and e represents the number of Rb bonded to Ar₂ and represents a number determined by subtracting (d + 2) from the number of substitutable substituents in Ar₂.
[3] The cyanate ester compound according to [2], wherein each X in the formula (1) is independently selected from the group consisting of
   a divalent organic group having 1 or more and 50 or less carbon atoms, the divalent organic group being represented by the following formula (3): 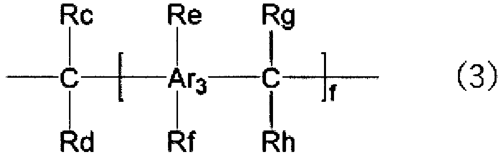 wherein each Ar₃ independently represents an aromatic ring, Rc, Rd, Rg, and Rh each independently represent a hydrogen atom, an alkyl group having 1 or more and 6 or less carbon atoms, or an aryl group having 6 or more and 12 or less carbon atoms, Re and Rf each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and f represents an integer of 0 or larger and 5 or smaller,
   a divalent organic group having 1 or more and 50 or less carbon atoms, the divalent organic group being represented by the following formula (4): 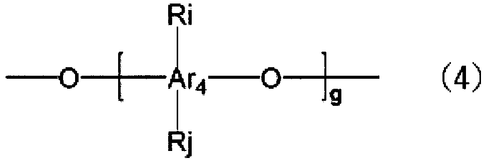 wherein each Ar₄ independently represents an aromatic ring, Ri and Rj each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and g represents an integer of 0 or larger and 5 or smaller, and
   divalent groups represented by the following formulas (5) to (14): 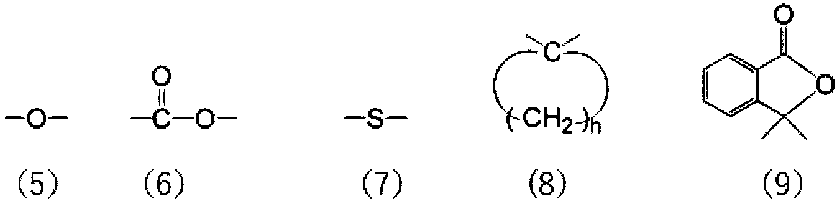 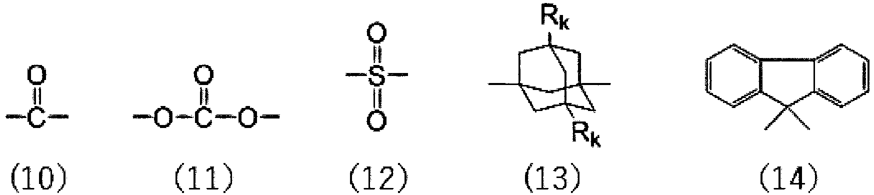 wherein h in the formula (8) represents an integer of 4 or larger and 7 or smaller, and each Rk in the formula (13) independently represents a hydrogen atom or an alkyl group having 1 or more and 6 or less carbon atoms.
[4] The cyanate ester compound according to any one of [1] to [3], wherein the cyanate ester compound is a compound represented by the following formula (15) or a compound represented by the following formula (16): 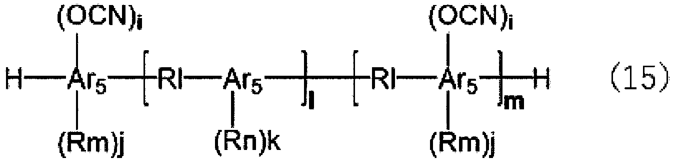
   wherein each Ar₅ independently represents an aromatic ring, each RI independently represents a methylene group, a methyleneoxy group, a methyleneoxymethylene group, or an oxymethylene group, or two or more of these groups linked to each other, Rm and Rn each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, i represents the number of cyanate groups bonded to Ar₅ and each independently represents an integer of 1 or larger and 3 or smaller, j represents the number of Rm bonded to Ar₅ and represents a number determined by subtracting (i + 2) from the number of substitutable substituents in Ar₅, k represents the number of Rn bonded to Ar₅ and represents a number determined by subtracting 2 from the number of substitutable substituents in Ar₅, I represents an integer of 1 or larger, m represents an integer of 1 or larger, and each repeating unit is arbitrarily arranged, and 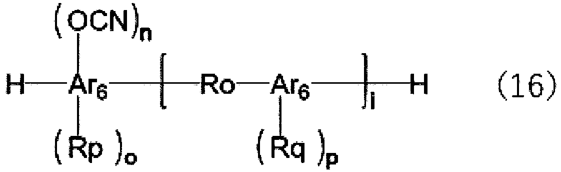
   wherein each Ar₆ independently represents an aromatic ring, each Ro independently represents a methylene group, a methyleneoxy group, a methyleneoxymethylene group, or an oxymethylene group, or two or more of these groups linked to each other, Rp and Rq each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, n represents the number of cyanate groups bonded to Ar₆ and is an integer of 2 or larger and 3 or smaller, o represents the number of Rp bonded to Ar₆ and represents a number determined by subtracting (n + 2) from the number of substitutable substituents in Ar₆, p represents the number of Rq bonded to Ar₆ and represents a number determined by subtracting 2 from the number of substitutable substituents in Ar₆, and i represents an integer of 1 or larger.
[5] The cyanate ester compound according to any one of [1] to [4], wherein the cyanate ester compound is a compound represented by the following formula (17): 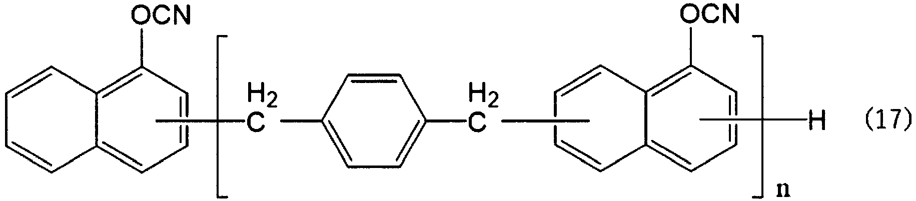 wherein n represents an integer of 1 or larger and 50 or smaller.
[6] The cyanate ester compound according to any one of [1] to [5], wherein the compound having a triazine ring is a compound having a structure represented by the following formula (18): 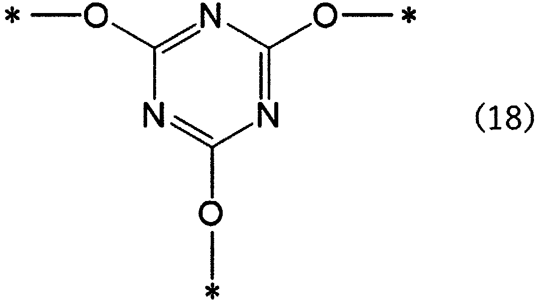 wherein * represents a binding site.
[7] A method for producing a cyanate ester compound according to any one of [1] to [6], containing: a starting material solution preparation step of preparing a first solution containing a hydroxy-substituted aromatic compound, a basic compound, and an organic solvent in a container, wherein the first solution is prepared while an inert gas is circulated at 0.1 L/hr or more and 20 L/hr or less per L of the container; and a cyanation step of bringing the first solution into contact with a second solution containing cyanogen halide, hydrogen halide, an organic solvent, and water, and performing cyanation through reaction to obtain a reaction liquid containing the cyanate ester compound.
[8] The method for producing a cyanate ester compound according to [7], wherein in the starting material solution preparation step, a temperature of the first solution is 5.0°C or lower.
[9] The method for producing a cyanate ester compound according to [7] or [8], wherein the cyanation step contains bringing the first solution into contact with the second solution to obtain a reaction liquid, and then pouring a third solution containing a basic compound and an organic solvent to the reaction liquid.
[10] The method for producing a cyanate ester compound according to any one of [7] to [9], wherein in the cyanation step, an amount of a starting material charged for the cyanogen halide is 0.5 mol or more and 5.0 mol or less based on 1 mol of a hydroxy group of the hydroxy-substituted aromatic compound.
[11] The method for producing a cyanate ester compound according to [7], wherein in the cyanation step, a temperature of the reaction liquid is 5.0°C or lower.
[12] A resin composition containing the cyanate ester compound according to any one of [1] to [6].
[13] A cured product obtained by curing the resin composition according to [12].

### Advantageous Effect of Invention

The present invention can provide a cyanate ester compound that facilitates controlling polymerization reaction at the time of cured product production and is suitably used in the production of a cured product having desired characteristics, and a method for producing the same, a resin composition, and a cured product.

### Description of Embodiments

Hereinafter, the mode for carrying out the present invention (hereinafter, referred to as the "present embodiment") will be described in detail. The present embodiment given below is an illustration for describing the present invention and does not intend to limit the present invention to the contents given below. The present invention can be carried out through appropriate changes or modifications made without departing from the spirit of the present invention.

In the present embodiment, the term "resin solid content" or "resin solid content in a resin composition" refers to resin components except for a filler, additives (silane coupling agent, wetting and dispersing agent, curing accelerator, and other components), and a solvent in the resin composition, unless otherwise specified. The term "100 parts by mass of the resin solid content" or "100 parts by mass in total of the resin solid content in a resin composition" means that the total of resin components except for a filler, additives (silane coupling agent, wetting and dispersing agent, curing accelerator, and other components), and a solvent in the resin composition is 100 parts by mass.

In the present specification, examples of the substituent include, but are not particularly limited to, halogen atoms such as a fluorine atom, a chlorine atom, a bromine atom, and an iodine atom, a hydroxy group, a cyano group, a nitro group, a thiol group, heterocyclic groups, linear aliphatic hydrocarbon groups, branched aliphatic hydrocarbon groups, cyclic aliphatic hydrocarbon groups, aryl groups, aralkyl groups, alkoxy groups, alkenyl groups, acyl groups, alkoxycarbonyl groups, alkyloyloxy groups, aryloyloxy groups, and alkylsilyl groups. For specific examples of these substituents, see, for example, examples of the groups described in the present specification.

### [Cyanate ester compound]

The cyanate ester compound of the present embodiment is a cyanate ester compound having two or more cyanate groups in a molecule, wherein a content of a compound having a triazine ring is 4.0% by area or less on HPLC area percentage.

The present embodiment can provide a cyanate ester compound that facilitates controlling polymerization reaction at the time of cured product production and is suitably used in the production of a cured product having desired characteristics, and a resin composition containing the cyanate ester compound. Use of the cyanate ester compound and the resin composition of the present embodiment can suitably control polymerization reaction at the time of curing and therefore facilitates producing a cured product having characteristics appropriate for a desired purpose. Although the reason for this is not certain, the present inventors have deduced the reason as follows.

Specifically, the compound having a triazine ring accelerates the polymerization of the cyanate ester compound. However, in the cyanate ester compound of the present embodiment, the content of the compound having a triazine ring is suitably controlled and suppressed so as to be 4.0% by area or less on HPLC area percentage. Therefore, polymerization reaction at the time of cured product production can be suitably controlled.

Hence, according to the deduction of the present inventors, the present embodiment can provide a cyanate ester compound that facilitates controlling polymerization reaction at the time of cured product production and is suitably used in the production of a cured product having desired characteristics, and a resin composition containing the cyanate ester compound. However, the reason is not limited thereto. The cyanate ester compound of the present embodiment can be suitably obtained by use of, for example, a production method described later.

The content of the compound having a triazine ring is preferably 3.0% by area or less, more preferably 2.0% by area or less, further preferably 1.5% by area or less, on HPLC area percentage because the resulting cyanate ester compound more facilitates controlling polymerization reaction at the time of cured product production and is more suitably used in the production of a cured product having desired characteristics. The lower limit is not particularly limited and is usually 0.5% by area or more, for example, in consideration of the lower limit of quantification in HPLC.

A method for measuring the content of the compound having a triazine ring involves measurement using a high performance liquid chromatograph (HPLC). Examples of such a measurement method include a method of measuring the content under conditions given below. For a specific measurement method, see the description of Examples.

Specifically, 2.0 g of a 2-butanone solution having a content of 50% by mass of the obtained cyanate ester compound is dissolved in 50 g of tetrahydrofuran (solvent) to obtain a solution. Analysis is conducted by high performance liquid chromatography after injection of 2.0 µL of the solution. The measurement conditions involve, for example, TSKgel ODS-120T manufactured by Tosoh Corp. (25 cm in length × 4.6 mm in inside diameter) used as a column, acetonitrile/water (80/20 volume ratio) used as a mobile phase, a flow rate of 1.0 mL/min., a detection wavelength of 274 nm, and a column temperature of 35°C. When the cyanate ester compound is, for example, a compound represented by the formula (17), a peak observed at a retention time (RT) of 1.5 minutes under the conditions is regarded as a peak derived from a compound having a triazine ring represented by the formula (18), and the content of the compound having a triazine ring in the cyanate ester compound is calculated from a peak area ratio thereof.

Examples of the compound having a triazine ring include compounds having a group represented by the following formula (19):

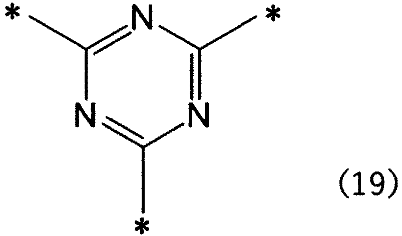

In the formula (19), "-*" represents a binding site.

These binding sites are each independently bonded to an organic group or a hydrogen atom. Specifically, the compound having a triazine ring of the present embodiment also encompasses triazine.

Examples of the organic group include groups containing one or more carbon atoms, and groups formed by removing one hydrogen atom from organic compounds. Examples of such an organic group include amino groups, alkyl groups, cycloalkyl groups, alkoxy groups, aryl groups, aralkyl groups, alkylcarbonyl groups, alkoxycarbonyl groups, and alkylcarbonyloxy groups. These groups may each have an ether bond or a carbonyl group.

In the compound having a triazine ring, when each binding site in the formula (19) is bonded to the organic group via an ether bond, the content of the compound having a triazine ring preferably falls within the range described above. The content of such a compound within the range described above tends to produce the cyanate ester compound that more facilitates controlling polymerization reaction at the time of cured product production, and is more suitably used in the production of a cured product having desired characteristics.

The content of a compound represented by the following formula (18) as the compound having a triazine ring preferably falls within the range described above because the resulting cyanate ester compound more facilitates controlling polymerization reaction at the time of cured product production and is more suitably used in the production of a cured product having desired characteristics.

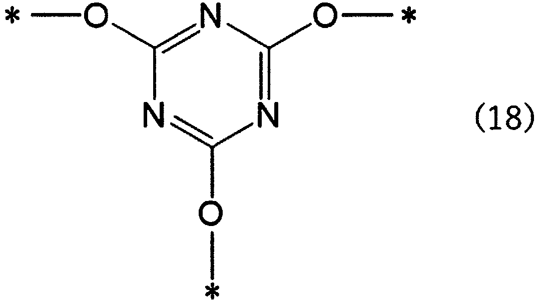

In the formula (18), "-*" represents a binding site.

In the present embodiment, the compound having a triazine ring is a by-product resulting from excessive progression of cyanation of a hydroxy-substituted aromatic compound in the presence of a basic compound. Therefore, the compound having a triazine ring has a triazine ring formed by the cyclization of cyanate groups in the presence of a basic compound. The binding site is bonded to the cyanate ester compound. When the cyanate ester compound has, for example, a structure where cyanate groups are bonded to an aromatic ring, the binding site is bonded to the aromatic ring in the cyanate ester compound. Specifically, an oxygen atom in "-*-O" in the formula (18) is an oxygen atom in a cyanate group.

The cyanate ester compound is preferably a compound represented by the following formula (1) or a compound represented by the following formula (2), more preferably a compound represented by the formula (1), because the resulting cyanate ester compound more facilitates controlling polymerization reaction at the time of cured product production and is more suitably used in the production of a cured product having desired characteristics.

### (Compound represented by formula (1))

The compound represented by the formula (1) will be shown below.

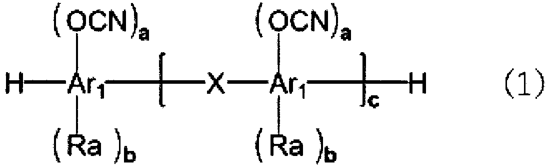

In the formula (1), each Ar₁ independently represents an aromatic ring, each Ra independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms, a represents the number of cyanate groups bonded to Ar₁ and each independently represents an integer of 1 or larger and 3 or smaller, b represents the number of Ra bonded to Ar₁ and each independently represents a number determined by subtracting (a + 2) from the number of substitutable substituents in Ar₁, c is an integer of 1 or larger and 50 or smaller, and each X independently represents a single bond, a divalent organic group having 1 or more and 50 or less carbon atoms in which a hydrogen atom is optionally replaced with a heteroatom, a divalent organic group having 1 or more and 10 or less nitrogen atoms, a carbonyl group, a carboxy group, a carbonyl dioxide group, a sulfonyl group, a divalent sulfur atom, or a divalent oxygen atom. Each group in the formula (1) may have a substituent.

In the formula (1), each Ar₁ independently represents an aromatic ring. Examples of the aromatic ring represented by Ar₁ include a phenyl group and a naphthyl group. Ar₁ is preferably a phenyl group because the resulting cyanate ester compound more facilitates controlling polymerization reaction at the time of cured product production and is more suitably used in the production of a cured product having desired characteristics.

Each Ra independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms. Ra is preferably a hydrogen atom or an alkenyl group having 2 or more and 6 or less carbon atoms because the resulting cyanate ester compound more facilitates controlling polymerization reaction at the time of cured product production and is more suitably used in the production of a cured product having desired characteristics.

Examples of the alkyl group having 1 or more and 6 or less carbon atoms include a methyl group, an ethyl group, a n-propyl group, an isopropyl group, a n-butyl group, an isobutyl group, a s-butyl group, a t-butyl group, a n-pentyl group, a neopentyl group, a n-hexyl group, a cyclopropyl group, a cyclobutyl group, a cyclopentyl group, and a cyclohexyl group. The alkyl group may be linear, branched, or cyclic.

Examples of the alkenyl group having 2 or more and 6 or less carbon atoms include a vinyl group, an allyl group, a butenyl group, a pentenyl group, and a hexenyl group. The alkenyl group having 2 or more and 6 or less carbon atoms is preferably an alkenyl group having 2 or more and 5 or less carbon atoms because the resulting cyanate ester compound more facilitates controlling polymerization reaction at the time of cured product production and is more suitably used in the production of a cured product having desired characteristics. The alkenyl group may be linear, branched, or cyclic.

Examples of the aryl group having 6 or more and 20 or less carbon atoms include a phenyl group and a naphthyl group.

Examples of the alkoxy group having 1 or more and 4 or less carbon atoms include a methoxy group, an ethoxy group, a propoxy group, and a butoxy group. The alkoxy group may be linear, branched, or cyclic.

a represents the number of cyanate groups bonded to Ar₁ and each independently represents an integer of 1 or larger and 3 or smaller. a is preferably an integer of 1 or larger and 2 or smaller, more preferably 1, because the resulting cyanate ester compound more facilitates controlling polymerization reaction at the time of cured product production and is more suitably used in the production of a cured product having desired characteristics.
b represents the number of Ra bonded to Ar₁ and each independently represents a number determined by subtracting (a + 2) from the number of substitutable substituents in Ar₁.
c is an integer of 1 or larger and 50 or smaller. c is preferably an integer of 1 or larger and 10 or smaller, more preferably an integer of 1 or larger and 5 or smaller, further preferably 1, because the resulting cyanate ester compound more facilitates controlling polymerization reaction at the time of cured product production and is more suitably used in the production of a cured product having desired characteristics.

Each X independently represents a single bond, a divalent organic group having 1 or more and 50 or less carbon atoms in which a hydrogen atom is optionally replaced with a heteroatom, a divalent organic group having 1 or more and 10 or less nitrogen atoms (-N-R-N- wherein R represents an organic group), a carbonyl group (-CO-), a carboxy group (-C(=O)O-), a carbonyl dioxide group (-OC(=O)O-), a sulfonyl group (-SO₂-), a divalent sulfur atom, or a divalent oxygen atom.

The divalent organic group having 1 or more and 50 or less carbon atoms in which a hydrogen atom is optionally replaced with a heteroatom is, for example, a linking group selected from the group consisting of divalent groups represented by the formulas (3) to (14).

Each X in the compound represented by the formula (1) is independently preferably selected from the group consisting of
a divalent organic group having 1 or more and 50 or less carbon atoms, the divalent organic group being represented by the following formula (3): 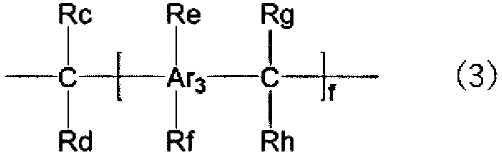 wherein each Ar₃ independently represents an aromatic ring, Rc, Rd, Rg, and Rh each independently represent a hydrogen atom, an alkyl group having 1 or more and 6 or less carbon atoms, or an aryl group having 6 or more and 12 or less carbon atoms, Re and Rf each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and f represents an integer of 0 or larger and 5 or smaller,
a divalent organic group having 1 or more and 50 or less carbon atoms, the divalent organic group being represented by the following formula (4): 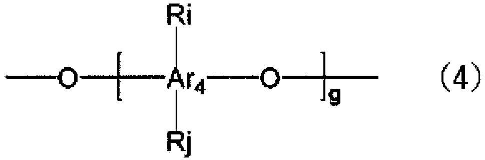 wherein each Ar₄ independently represents an aromatic ring, Ri and Rj each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and g represents an integer of 0 or larger and 5 or smaller, and
divalent groups represented by the following formulas (5) to (14): 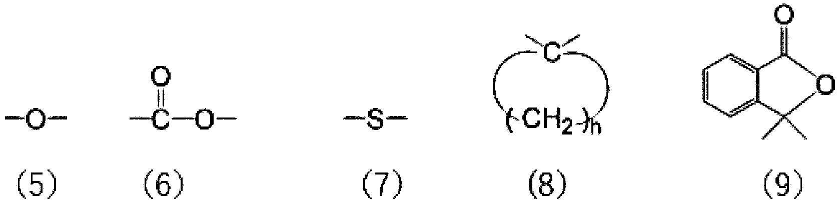 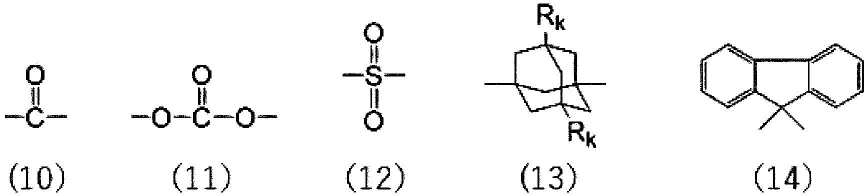 wherein h in the formula (8) represents an integer of 4 or larger and 7 or smaller, and each Rk in the formula (13) independently represents a hydrogen atom or an alkyl group having 1 or more and 6 or less carbon atoms, because the resulting cyanate ester compound more facilitates controlling polymerization reaction at the time of cured product production and is more suitably used in the production of a cured product having desired characteristics.

In the formulas (3) and (4), examples of the aromatic ring represented by each of Ar₃ and Ar₄ can include the same as those listed in Ar₁. In the formulas (3) and (4), examples of the alkyl group having 1 or more and 6 or less carbon atoms or the aryl group having 6 or more and 12 or less carbon atoms represented by each of Rc, Rd, Rg, and Rh as well as the alkyl group having 1 or more and 6 or less carbon atoms, the aryl group having 6 or more and 12 or less carbon atoms, or the alkoxy group having 1 or more and 4 or less carbon atoms represented by each of Re, Rf, Ri, and Rj can include the same as those listed in Ra. Each group in the formulas (3) and (4) may have a substituent.

Examples of the compound represented by the formula (1) include bisphenol A-type cyanate, bisphenol E-type cyanate, and diallyl bisphenol A-type cyanate.

The compound represented by the formula (1) is preferably a compound represented by the following formula (15) because the compound more facilitates controlling polymerization reaction at the time of cured product production and is more suitably used in the production of a cured product having desired characteristics.

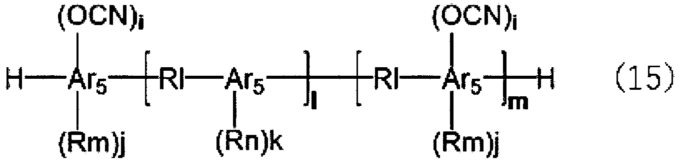

In the formula (15), each Ar₅ independently represents an aromatic ring, each RI independently represents a methylene group, a methyleneoxy group, a methyleneoxymethylene group, or an oxymethylene group, or two or more of these groups linked to each other, Rm and Rn each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, i represents the number of cyanate groups bonded to Ar₅ and each independently represents an integer of 1 or larger and 3 or smaller, j represents the number of Rm bonded to Ar₅ and represents a number determined by subtracting (i + 2) from the number of substitutable substituents in Ar₅, k represents the number of Rn bonded to Ar₅ and represents a number determined by subtracting 2 from the number of substitutable substituents in Ar₅, I represents an integer of 1 or larger, m represents an integer of 1 or larger, and each repeating unit is arbitrarily arranged. Each group in the formula (15) may have a substituent.

In the formula (15), examples of the aromatic ring represented by Ar₅ can include the same as those listed in Ar₁. In the formula (15), examples of the alkyl group having 1 or more and 6 or less carbon atoms, the aryl group having 6 or more and 12 or less carbon atoms, and the alkoxy group having 1 or more and 4 or less carbon atoms represented by each of Rm and Rn can include the same as those listed in Ra. I represents an integer of 1 or larger and is preferably an integer of 1 or larger and 10 or smaller, more preferably an integer of 1 or larger and 5 or smaller. m represents an integer of 1 or larger and is preferably an integer of 1 or larger and 10 or smaller, more preferably an integer of 1 or larger and 5 or smaller.

### (Compound represented by formula (2))

The compound represented by the formula (2) will be shown below.

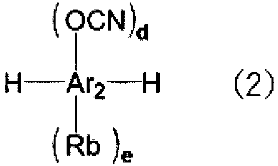

In the formula (2), Ar₂ represents an aromatic ring, each Rb independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms, d represents the number of cyanate groups bonded to Ar₂ and is an integer of 2 or larger and 3 or smaller, and e represents the number of Rb bonded to Ar₂ and represents a number determined by subtracting (d + 2) from the number of substitutable substituents in Ar₂. Each group in the formula (2) may have a substituent.

In the formula (2), examples of the aromatic ring represented by Ar₂ can include the same as those listed in Ar₁. In the formula (2), examples of the alkenyl group having 2 or more and 6 or less carbon atoms, the aryl group having 6 or more and 12 or less carbon atoms, or the alkoxy group having 1 or more and 4 or less carbon atoms represented by Rb can include the same as those listed in Ra.

The cyanate ester compound of the present embodiment is preferably a compound represented by the following formula (16):

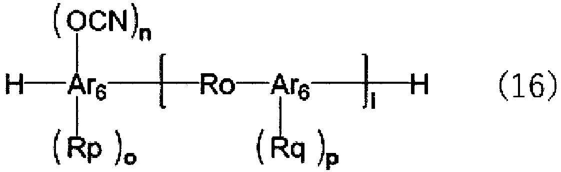

In the formula (16), each Ar₆ independently represents an aromatic ring, each Ro independently represents a methylene group, a methyleneoxy group, a methyleneoxymethylene group, or an oxymethylene group, or two or more of these groups linked to each other, Rp and Rq each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, n represents the number of cyanate groups bonded to Ar₆ and is an integer of 2 or larger and 3 or smaller, o represents the number of Rp bonded to Ar₆ and represents a number determined by subtracting (n + 2) from the number of substitutable substituents in Ar₆, p represents the number of Rq bonded to Ar₆ and represents a number determined by subtracting 2 from the number of substitutable substituents in Ar₆, and i represents an integer of 1 or larger. Each group in the formula (16) may have a substituent.

In the formula (16), examples of the aromatic ring represented by Ar₆ can include the same as those listed in Ar₁. In the formula (16), examples of the alkyl group having 1 or more and 6 or less carbon atoms, the aryl group having 6 or more and 12 or less carbon atoms, and the alkoxy group having 1 or more and 4 or less carbon atoms represented by each of Rp and Rq can include the same as those listed in Ra. i represents an integer of 1 or larger and is preferably an integer of 1 or larger and 10 or smaller, more preferably an integer of 1 or larger and 5 or smaller.

The compound represented by the formula (1) is preferably a compound represented by the following formula (17) because the compound further facilitates controlling polymerization reaction at the time of cured product production and is further suitably used in the production of a cured product having desired characteristics.

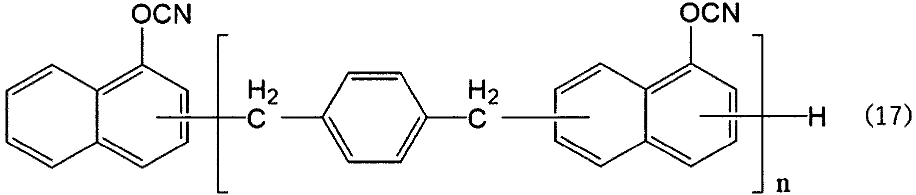

In the formula (17), n represents an integer of 1 or larger and 50 or smaller. n is preferably an integer of 1 or larger and 10 or smaller because the resulting cyanate ester compound still more facilitates controlling polymerization reaction at the time of cured product production and is still more suitably used in the production of a cured product having desired characteristics.

### [Method for producing cyanate ester compound]

The method for producing the cyanate ester compound of the present embodiment contains: a starting material solution preparation step of preparing a first solution (hereinafter, also referred to as a "solution 1") containing a hydroxy-substituted aromatic compound, a basic compound (hereinafter, also referred to as a "basic compound 1"), and an organic solvent (hereinafter, also referred to as an "organic solvent 1") in a container, wherein the first solution is prepared while an inert gas is circulated at 0.1 L/hr or more and 20 L/hr or less per L of the container; and a cyanation step of bringing the first solution into contact with a second solution (hereinafter, also referred to as a "solution 2") containing cyanogen halide, hydrogen halide, a second organic solvent (hereinafter, also referred to as an "organic solvent 2"), and water, and performing cyanation through reaction to obtain a reaction liquid containing the cyanate ester compound (hereinafter, also referred to as a "cyanation step"). However, the organic solvent 2 may be the same as or different from the organic solvent 1.

Through such specific steps, the cyanate ester compound can be preferably produced which has a content of 4.0% by area or less of the compound having a triazine ring on HPLC area percentage, facilitates controlling polymerization reaction at the time of cured product production, and is suitably used in the production of a cured product having desired characteristics. Although the reason for this is not certain, the present inventors have deduced the reason as follows.

Specifically, the production method of the present embodiment contains, before the cyanation step, the step of preparing a solution 1 containing a hydroxy-substituted aromatic compound, a basic compound 1, and an organic solvent 1 in a container while an inert gas is circulated at 0.1 L/hr or more and 20 L/hr or less per L of the container, as the starting material solution preparation step. In the hydroxy-substituted aromatic compound obtained through this step, oxidation reaction that converts a hydroxy group to ketone is suitably suppressed. The cyanation step for the cyanate ester compound employs, as a starting material solution, a solution containing a hydroxy-substituted aromatic compound prevented from being oxidized into ketone. Therefore, in the cyanation step, excessive cyanation of the hydroxy-substituted aromatic compound is suitably suppressed, and reaction that converts a hydroxy group in the hydroxy-substituted aromatic compound to a cyanate group can progress more smoothly. This enables the production of the compound having a triazine ring as a by-product to be suitably controlled.

Hence, according to the deduction of the present inventors, the production method of the present embodiment can preferably produce a cyanate ester compound that has a content of 4.0% by area or less of the compound having a triazine ring on HPLC area percentage, facilitates controlling polymerization reaction at the time of cured product production, and is suitably used in the production of a cured product having desired characteristics. However, the reason is not limited thereto.

First, each step in the method for producing the cyanate ester compound will be described. Each component to be subjected to the starting material solution preparation step, the cyanation step, and the like will be mentioned later.

### [Starting material solution preparation step]

The method for producing the cyanate ester compound of the present embodiment contains a starting material solution preparation step of preparing a solution 1 containing a hydroxy-substituted aromatic compound, a basic compound 1, and an organic solvent 1 in a container, wherein the solution 1 is prepared while an inert gas is circulated at 0.1 L/hr or more and 20 L/hr or less per L of the container. The organic solvent 1 may be the same as or different from the organic solvent 2 and is preferably the same thereas.

A known container can be used as the container, depending on the properties of starting materials, etc. as long as the container enables an inert gas to be circulated at 0.1 L/hr or more and 20 L/hr or less per L of the container. In the present embodiment, the container is preferably an openable container because starting materials are prepared while an inert gas is circulated. Examples of such a container include containers having an openable and closable lid, and containers in which supply piping and a valve for inert gas circulation are installed in a portion of the container and a vent line (discharge piping) is installed on the exit side.

The container is preferably a container having a function that can stir a solution. Examples of such a function that can stir a solution include stirring blades, stirrers, paddles, ribbons, screws, and mixers.

Examples of the material of the container include: glass; metal materials such as stainless steel, nickel, Monel(R), and Hastelloy(R); fluorine-containing resins such as polytetrafluoroethylene (PTFE); and plastics such as polyethylene terephthalate. The container may be lined at its surface or inner face with a resin or the like. Examples of such a resin include fluorine-containing resins such as polytetrafluoroethylene (PTFE), tetrafluoroethylene-perfluoroalkyl vinyl ether copolymers (PFA), and tetrafluoroethylene-hexafluoropropylene copolymers (FEP), and olefin resins such as polypropylene.

The container is preferably a container having glass or a container having stainless steel. Use of such a container can preferably produce the cyanate ester compound that has the content of the compound having a triazine ring within the range described above, facilitates controlling polymerization reaction at the time of cured product production, and is suitably used in the production of a cured product having desired characteristics. In addition to the effects described above, the container is more preferably a container having stainless steel because of high solvent resistance and inexpensiveness.

Examples of the shape of the container include spherical, prolate ellipsoid, horizontal cylindrical, sloped cylindrical, V, coned, cubic, and flask shapes.

Specific examples of the container include glass containers such as flask, bottles, and vials, and known vessels such as reaction vessels.

The capacity of the container can be appropriately set depending on the properties of starting materials, etc. as long as the capacity enables an inert gas to be circulated at 0.1 L/hr or more and 20 L/hr or less per L of the container. The capacity of the container is preferably 0.1 L or more and 30000 L or less. Use of such a container can preferably produce the cyanate ester compound that has the content of the compound having a triazine ring within the range described above, facilitates controlling polymerization reaction at the time of cured product production, and is suitably used in the production of a cured product having desired characteristics.

In the starting material solution preparation step, the gas flow rate of the inert gas is preferably 0.2 L/hr or more and 18 L/hr or less, more preferably 0.3 L/hr or more and 17 L/hr or less, per L of the container. In the starting material solution preparation step, the inert gas can be circulated so as to create an inert gas atmosphere in the container. The inert gas may be circulated in contact with the upper face (liquid surface) of starting materials, or the inert gas may be bubbled into starting materials. Before preparation of the starting material solution, the inert gas is preferably circulated in advance so as to create an inert gas atmosphere in the container.

Examples of the inert gas include nitrogen, helium, and argon. The inert gas is preferably nitrogen. Use of nitrogen can preferably produce the cyanate ester compound that has the content of the compound having a triazine ring within the range described above, facilitates controlling polymerization reaction at the time of cured product production, and is suitably used in the production of a cured product having desired characteristics.

In the starting material solution preparation step, the temperature of the solution 1 containing the hydroxy-substituted aromatic compound, the basic compound 1, and the organic solvent 1 is preferably 5.0°C or lower, more preferably -20°C or higher and 3.0°C or lower, further preferably -10°C or higher and 1.0°C or lower. The temperature of the solution within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

In the starting material solution preparation step, the internal pressure of the container is not particularly limited as long as a solution obtained in the desired starting material solution preparation step is obtained at the pressure.

In the starting material solution preparation step, the time for which the inert gas is circulated in the container is preferably 1 minute or longer and 20 hours or shorter, more preferably 3 minutes or longer and 10 hours or shorter. In the present embodiment, the solution obtained in the starting material solution preparation step is subjected as a starting material to the next cyanation step. Therefore, the inert gas is preferably circulated in the container until then. This tends to enable the cyanate ester compound to be more preferably produced which has the content of the compound having a triazine ring within the range described above, facilitates controlling polymerization reaction at the time of cured product production, and is suitably used in the production of a cured product having desired characteristics.

In the starting material solution preparation step or in the preparation of the solution obtained in the starting material solution preparation step, the amount of the organic solvent 1 blended is preferably an amount that can dissolve the basic compound 1 and the hydroxy-substituted aromatic compound. The amount of the organic solvent 1 blended is usually 50% by mass or more and 90% by mass or less based on 100% by mass of the solution (starting material).

In the starting material solution preparation step or in the preparation of the solution obtained in the starting material solution preparation step, the amount of the basic compound 1 blended is preferably 0.1 mol or more and 8.0 mol or less, more preferably 0.5 mol or more and 3.0 mol or less, based on 1 mol of a hydroxy group of the hydroxy-substituted aromatic compound. The blending ratio between the basic compound 1 and the hydroxy-substituted aromatic compound within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

### [Cyanation step]

The method for producing the cyanate ester compound contains a cyanation step of bringing the solution 1 into contact with a solution 2 and performing cyanation through reaction to obtain a reaction liquid containing the cyanate ester compound. Such a specific step used as the cyanation step can more preferably produce the cyanate ester compound that has the content of the compound having a triazine ring within the range described above, facilitates controlling polymerization reaction at the time of cured product production, and is suitably used in the production of a cured product having desired characteristics.

Examples of the method for bringing the solution 1 into contact with the solution 2 for reaction include a method of stirring the cyanogen halide contained in the solution 2 and the hydroxy-substituted aromatic compound contained in the solution 1 in the presence of the basic compound contained in the solution 1 so that the components are brought into contact with each other for reaction.

In the cyanation step, the operation method of bringing the solution 1 into contact with the solution 2 can be performed by a semi-batch format or a continuous circulation format. Specifically, examples of the contact operation method include a method (a) of adding the solution 1 dropwise to the solution 2 with stirring and mixing, a method (b) of adding the solution 2 dropwise to the solution 1 with stirring and mixing, and a method (c) of supplying a portion of the solution 2 and a portion of the solution 1 continuously or intermittently and alternately or simultaneously to a reaction container. In these methods, each solution may be added dropwise in appropriately divided portions. Examples of the method (a) include a method of adding dropwise the solution 1 in divided portions to the solution 2 with stirring and mixing. The number of divided portions for dropwise addition is, for example, usually 2 or more and 5 or less.

Among the methods (a) to (c), the method (a) is preferred. Use of the method (a) as the contact operation method can more preferably produce the cyanate ester compound that has the content of the compound having a triazine ring within the range described above, facilitates controlling polymerization reaction at the time of cured product production, and is suitably used in the production of a cured product having desired characteristics.

In the cyanation step, the reaction time is not particularly limited. The dropwise addition time in the method (a) or the method (b) as the contact operation or the contact time in the method (c) is preferably 1 minute or longer and 20 hours or shorter, more preferably 3 minutes or longer and 10 hours or shorter. It is preferred to then perform stirring while the reaction temperature is maintained for 10 minutes or longer and 10 hours or shorter. The reaction time falls within the range described above, whereby the cyanate ester compound of interest tends to be obtained economically and industrially more efficiently. For the stirring method, see the following description.

Examples of the stirring method include methods using a known stirring machine such as a stirring blade, a stirrer, a paddle, a ribbon, a screw, or a mixer. For the stirring time, see the reaction time described above. The stirring time is preferably 1 minute or longer and 20 hours or shorter, more preferably 3 minutes or longer and 10 hours or shorter.

In the cyanation step, the temperature of the reaction liquid is preferably 5.0°C or lower, more preferably -20°C or higher and 3.0°C or lower, further preferably - 10°C or higher and 2.0°C or lower. The temperature of the reaction liquid within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

In the cyanation step, the internal pressure of a container is not particularly limited as long as a reaction liquid containing the desired cyanate ester compound is obtained at the pressure. The reaction system may be ventilated, if necessary, with an inert gas such as nitrogen, helium, or argon.

The content of the organic solvent 1 in the solution 1 for use in the cyanation step is preferably an amount that can dissolve the basic compound and the hydroxy-substituted aromatic compound. The content of the organic solvent 1 is usually 50% by mass or more and 90% by mass or less based on 100% by mass of the solution 1.

The content of the basic compound 1 in the solution 1 for use in the cyanation step is preferably 0.1 mol or more and 8.0 mol or less, more preferably 0.5 mol or more and 3.0 mol or less, based on 1 mol of a hydroxy group of the hydroxy-substituted aromatic compound. The content ratio between the basic compound 1 and the hydroxy-substituted aromatic compound within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

For the amounts of the cyanogen halide and the hydrogen halide blended into the solution 2, see the amount of the hydrogen halide used and the amount of a starting material charged for the cyanogen halide mentioned later. The water is preferably in an amount that can dissolve the hydrogen halide. In the solution 2, the mass ratio of the water to the organic solvent 2 (water/organic solvent 2) is not particularly limited and is preferably 1/100 to 100/1, more preferably 1/10 to 10/1, further preferably 1/5 to 5/1.

The cyanation step is more preferably a cyanation step of bringing the solution 2 into contact with the solution 1 containing the hydroxy-substituted aromatic compound for cyanation to obtain a reaction liquid containing the cyanate ester compound, and then pouring a third solution (hereinafter, also referred to as a "solution 3") containing a basic compound (hereinafter, also referred to as a "basic compound 2") and an organic solvent (hereinafter, also referred to as an "organic solvent 3") to the reaction liquid. The basic compound 2 may be the same as or different from the basic compound 1 and is preferably the same thereas. The organic solvent 3 may be the same as or different from the organic solvent 1 and is preferably the same thereas. The organic solvent 3 may be the same as or different from the organic solvent 2 and is preferably the same thereas. After the reaction liquid is obtained, the solution 3 is added to the reaction liquid. This tends to enable a highly pure cyanate ester compound to be obtained at a high yield.

The basic compound 2 and the organic solvent 3 contained in the solution 3 are preferably the same components as or from the basic compound 1 and the organic solvent 1, respectively, contained in the solution 1. Such a specific step used as the cyanation step can further preferably produce the cyanate ester compound that has the content of the compound having a triazine ring within the range described above, facilitates controlling polymerization reaction at the time of cured product production, and is suitably used in the production of a cured product having desired characteristics.

The amount of the basic compound 2 blended into the solution 3 is preferably 0.05 mol or more and 2.5 mol or less, more preferably 0.1 mol or more and 2.3 mol or less, based on 1 mol of a hydroxy group of the hydroxy-substituted aromatic compound. The amount of the basic compound 2 used within the range described above tends to enable the cyanate ester compound to be further preferably produced at a high yield, which has the content of the compound having a triazine ring within the range described above, facilitates controlling polymerization reaction at the time of cured product production, and is suitably used in the production of a cured product having desired characteristics.

In the solution 3, the organic solvent 3 is preferably in an amount that can dissolve the basic compound 2. The content of three organic solvent 3 is usually 30% by mass or more and 90% by mass or less based on 100% by mass of the solution 3.

For the temperature in adding the solution 3 to the reaction liquid, see the temperature of the reaction liquid described above. The temperature in adding the solution 3 to the reaction liquid is preferably the same temperature as that of the reaction liquid described above because a highly pure cyanate ester compound tends to be obtained at a high yield.

The internal pressure of the container in adding the solution 3 to the reaction liquid is not particularly limited as long as the desired solution is obtained at the pressure. The system may be ventilated, if necessary, with an inert gas such as nitrogen, helium, or argon.

The time for which the solution 3 is added to the reaction liquid is preferably 1 minute or longer and 20 hours or shorter, more preferably 1.5 minutes or longer and 10 hours or shorter. It is preferred to then perform stirring while the same temperature as that in adding the solution 3 to the reaction liquid is maintained for 10 minutes or longer and 10 hours or shorter. The time for which the solution 3 is added to the reaction liquid falls within the range described above, whereby the cyanate ester compound of interest tends to be obtained economically and industrially more efficiently.

### [Other steps]

After the cyanation step or the step of pouring the solution 3 to the reaction liquid, the cyanate ester compound of interest can be isolated by performing a usual aftertreatment operation and, if desired, a separation operation and/or a purification operation. Specifically, the cyanogen halide and the organic solvent are distilled off by separating a solution containing an organic phase, which contains the cyanate ester compound, and an aqueous phase, followed by the heating and distillation of the organic phase thus separated. Then, an organic solvent is further added to the residue, which can then be washed with water and concentrated to precipitate or crystal-precipitate the cyanate ester compound of interest. Alternatively, after washing with water and concentration, the solvent in the organic phase may be replaced with a solvent in which the cyanate ester compound is insoluble or poorly soluble to precipitate or crystal-precipitate the cyanate ester compound. For washing, an acidic aqueous solution such as dilute hydrochloric acid may be used for removing an excess of amines. In order to remove moisture from the thoroughly washed organic phase, a drying operation may be performed by a general method using sodium sulfate, magnesium sulfate, or the like.

For concentration and solvent replacement, the organic solvent is preferably distilled off under reduced pressure and at a temperature of 90°C or lower because the polymerization of the cyanate ester compound is more suitably suppressed. The precipitation or the crystal precipitation can be performed by adding dropwise a solvent having low solubility for the cyanate ester compound into the solution containing the cyanate ester compound, or adding dropwise the solution containing the cyanate ester compound into a solvent having low solubility for the cyanate ester compound. Examples of the solvent having low solubility for the cyanate ester compound include: ether solvents; hydrocarbon solvents such as hexane; and alcohol solvents.

In order to wash the obtained crude product, the concentrate of the reaction liquid or the precipitates or the precipitated crystals may be washed with the solvent having low solubility for the cyanate ester compound. The crystals obtained by the concentration of the reaction liquid may be redissolved and then recrystallized. The crystal precipitation may be performed by the simple concentration or cooling of the reaction liquid.

The purity of the obtained cyanate ester compound can be analyzed by, for example, liquid chromatography or FT-IR (Fourier transform infrared spectroscopy). Volatile components such as by-products (e.g., dialkyl cyanamide) or residual solvents in the cyanate ester compound can be quantitatively analyzed by gas chromatography. Halide remaining in the cyanate ester compound can be identified using a liquid chromatography-mass spectrometer and can be quantitatively analyzed by potentiometric titration using a silver nitrate solution or by ion chromatography after decomposition by a combustion method. The polymerization reactivity of the cyanate ester compound can be evaluated on the basis of the time to gel by a hot plate method or a torque measurement method.

Next, each component to be subjected to the cyanation step will be described.

### (Hydrogen halide)

In the cyanation step, the hydrogen halide is used for adjusting pH and suppressing the production of impurities as a by-product.

In the cyanation step, the pH of the reaction liquid is preferably less than 7.0, more preferably 6.5 or less, further preferably 6.0 or less. The reaction performed with the pH of the reaction liquid kept in a state of less than 7.0 can more preferably produce the cyanate ester compound that has the content of the compound having a triazine ring within the range described above, facilitates controlling polymerization reaction at the time of cured product production, and is suitably used in the production of a cured product having desired characteristics.

Examples of the method for adding the hydrogen halide include a method of adding the hydrogen halide to the solution 1 in advance before cyanation, and a method of adding the hydrogen halide to the solution 1 while appropriately measuring pH with a pH meter. In this respect, for example, an inorganic acid such as nitric acid, sulfuric acid, or phosphoric acid, or an organic acid such as acetic acid, lactic acid, or propionic acid may be used as an acid, in addition to the hydrogen halide.

Examples of the hydrogen halide include hydrogen fluoride, hydrogen chloride, hydrogen bromide, and hydrogen iodide. The hydrogen halide may be an acidic aqueous solution such as hydrofluoric acid or hydrochloric acid. One of these hydrogen halides can be used singly, or two or more thereof can be used in combination.

The hydrogen halide is preferably hydrochloric acid because the cyanate ester compound can be more preferably produced which has the content of the compound having a triazine ring within the range described above, facilitates controlling polymerization reaction at the time of cured product production, and is suitably used in the production of a cured product having desired characteristics. When the hydrogen halide is hydrochloric acid, its concentration in the reaction liquid is preferably, for example, 1.0% or more and 10% or less. Use of the hydrochloric acid having such a concentration tends to produce highly pure cyanate ester.

The amount of the hydrogen halide used is preferably 0.5 mol or more and 5.0 mol or less, more preferably 1.0 mol or more and 3.5 mol or less, based on 1 mol of a hydroxy group of the hydroxy-substituted aromatic compound. The amount of the hydrogen halide used within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

### (Organic solvent)

A generally known solvent can be used as the organic solvent for use in each of the organic solvents 1 to 3 as long as the solvent is immiscible with water and inert to cyanation reaction. Examples of such an organic solvent include: halogenated hydrocarbon solvents such as dichloromethane, chloroform, carbon tetrachloride, dichloroethane, trichloroethane, chlorobenzene, and bromobenzene; aliphatic solvents such as n-hexane, cyclohexane, and isooctane; aromatic solvents such as benzene, toluene, xylene, and ethylbenzene; ketone solvents such as methyl ethyl ketone, cyclohexanone, cyclopentanone, and methyl isobutyl ketone; nitrile solvents such as benzonitrile; nitro solvents such as nitrobenzene; ether solvents such as diethyl ether, diisopropyl ether, and tetrahydrofuran; and ester solvents such as ethyl acetate and ethyl benzoate. One of these organic solvents can be used singly, or two or more thereof can be used in combination. Among them, the organic solvent is preferably a halogenated hydrocarbon solvent having 1 or more and 2 or less carbon atoms, such as dichloromethane, chloroform, carbon tetrachloride, dichloroethane, or trichloroethane.

In the present embodiment, an organic solvent other than those described above, for example, a solvent miscible with water, may be used as a mixture with the organic solvent described above as long as the advantageous effects of the present invention are exerted. Examples of such an organic solvent include acetone, dimethyl cellosolve, diglyme, tetraethylene glycol dimethyl ether, methanol, ethanol, isopropanol, methyl cellosolve, propylene glycol monomethyl ether, N,N-dimethylformamide, N-methylpyrrolidone, 1,3-dimethyl-2-imidazolidone, dimethyl sulfoxide, and acetonitrile.

### (Water)

Examples of the water include tap water, distilled water, and deionized water. Among them, distilled water or deionized water having a small content of impurities is preferably used from the viewpoint of efficiently obtaining the cyanate ester compound of interest.

The total amount of the solvents used in the cyanation step, i.e., the total amount of the organic solvents 1 to 3 and the water, is preferably 1.0 part by mass or more and 200 parts by mass or less, more preferably 1.5 parts by mass or more and 150 parts by mass or less, further preferably 2.5 parts by mass or more and 100 parts by mass or less, based on 1 part by mass of the hydroxy-substituted aromatic compound. The total amount of the solvents within the range described above tends to uniformly dissolve the hydroxy-substituted aromatic compound and more improve the production efficiency of the cyanate ester compound.

### (Cyanogen halide)

In the cyanation step, the cyanogen halide is used for cyanating the hydroxy-substituted aromatic compound.

Examples of the cyanogen halide include cyanogen fluoride, cyanogen chloride, cyanogen bromide, and cyanogen iodide. The cyanogen halide is preferably cyanogen chloride because the cyanate ester compound can be more preferably produced which has the content of the compound having a triazine ring within the range described above, facilitates controlling polymerization reaction at the time of cured product production, and is suitably used in the production of a cured product having desired characteristics.

The amount of a starting material charged for the cyanogen halide is preferably 0.5 mol or more and 5.0 mol or less, more preferably 1.0 mol or more and 3.5 mol or less, based on 1 mol of a hydroxy group of the hydroxy-substituted aromatic compound. The amount of the cyanogen halide used within the range described above tends to enable the cyanate ester compound to be more preferably produced which has the content of the compound having a triazine ring within the range described above, facilitates controlling polymerization reaction at the time of cured product production, and is suitably used in the production of a cured product having desired characteristics.

### (Hydroxy-substituted aromatic compound)

Examples of the hydroxy-substituted aromatic compound include aromatic compounds having at least one phenolic hydroxy group. Examples of such a hydroxy-substituted aromatic compound include phenolic resins having a polynaphthylene ether structure, and compounds represented by the formula (27). Examples of the compound represented by the formula (27) include (27a) a 1-naphthol aralkyl resin, (27b) a phenolic resin having an adamantane structure, and (27c) compounds other than these resins (27a) and (27b) (hereinafter, also referred to as "other hydroxy-substituted aromatic compounds"). One of these hydroxy-substituted aromatic compounds can be used singly, or two or more thereof can be used in combination.

### Phenolic resin having polynaphthylene ether structure

Examples of the phenolic resin having a polynaphthylene ether structure include resins having a polynaphthylene ether structure where a naphthalene ring is bonded to another naphthalene ring via an oxy group, and phenolic hydroxy groups on the naphthalene rings. The total number of naphthalene rings per molecule is preferably 2 or more and 8 or less. The presence of the polynaphthylene ether structure promotes the formation of char (carbonization residue) at the time of combustion of a cured product obtained from cyanate ester and exerts excellent flame retardancy while heat resistance is also favorable.

The number of oxy groups bonded per naphthalene ring is preferably 1 or more and 3 or less, more preferably 2, from the viewpoint of the fluidity of the phenolic resin having a polynaphthylene ether structure. In this respect, the binding positions of the oxy groups on the naphthalene ring are preferably 1,3-positions, 1,6-positions, 1,7-positions, 1,8-positions, 2,3-positions, or 2,7-positions. Among them, the binding positions of the oxy groups on the naphthalene ring are more preferably 1,6-positions or 2,7-positions from the viewpoint of easy production, and further preferably 2,7-positions from the viewpoint of excellent balance between fluidity and flame retardancy. As for a substituent other than the oxy groups on the naphthalene ring, the naphthalene ring is preferably free from the substituent from the viewpoint of a flame retardant effect.

The phenolic resin having a polynaphthylene ether structure may have a molecular structure where a plurality of naphthalene rings directly form a bond.

The phenolic resin having a polynaphthylene ether structure is, for example, one or more members selected from the group consisting of a compound represented by the formula (21) given below, and compounds represented by the formulas (23) to (26) given below as disclosed in Japanese Patent No. 4259536. A commercially available product may be used as the phenolic resin having a polynaphthylene ether structure. Examples of such a commercially available product include EXB-6000 from DIC Corp.

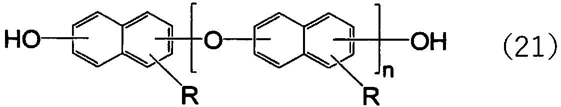

In the formula (21), each R independently represents a hydrogen atom, an aryl group or an alkyl group such as a benzyl group, or a group represented by the following formula (22), and n is an integer of 1 or larger and 20 or smaller, more preferably an integer of 1 or larger and 10 or smaller. Each group in the formula (21) may have a substituent.

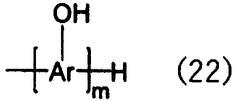

In the formula (22), each Ar independently represents an aryl group such as a phenylene group or a naphthylene group, and m is an integer of 1 or 2.

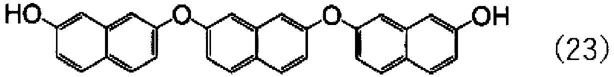

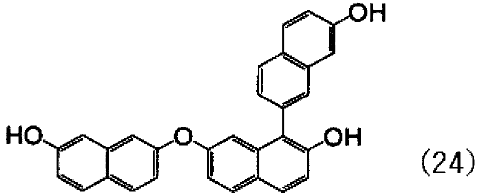

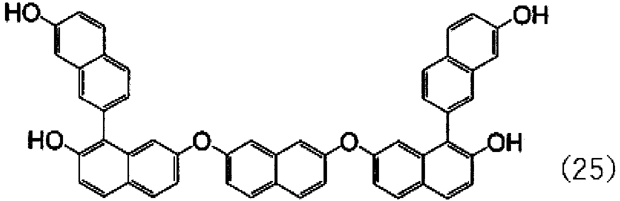

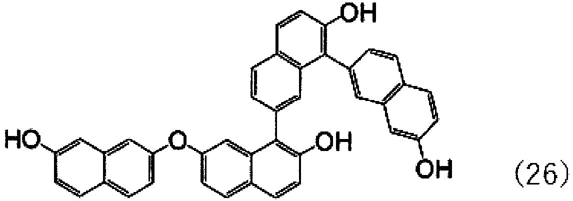

The phenolic resin having a polynaphthylene ether structure can be obtained through dehydration condensation reaction. The dehydration condensation reaction is reaction through which, before the cyanation step, a polyvalent hydroxynaphthalene compound having two or more phenolic hydroxy groups in one molecule is subjected to dehydration condensation reaction in the presence of a basic catalyst to obtain a hydroxy-substituted aromatic compound. Examples of the resulting hydroxy-substituted aromatic compound include phenolic resins having a structure where a naphthalene ring is bonded to another naphthalene ring via an oxygen atom (hereinafter, also referred to as an "oxy group") (hereinafter, also referred to as a "polynaphthylene ether structure").

Examples of the polyvalent hydroxynaphthalene compound for use in the dehydration condensation reaction include: dihydroxynaphthalene such as 1,3-dihydroxynaphthalene, 1,6-dihydroxynaphthalene, 1,7-dihydroxynaphthalene, 1,8-dihydroxynaphthalene, 2,3-dihydroxynaphthalene, and 2,7-dihydroxynaphthalene; trihydroxynaphthalene such as 1,2,3-trihydroxynaphthalene; and compounds having an alkyl group having 1 or more and 4 or less carbon atoms or a phenyl group as a substituent on the aromatic rings of these compounds. One of these polyvalent hydroxynaphthalene compounds can be used singly, or two or more thereof can be used in combination.

Examples of the basic catalyst for use in the dehydration condensation reaction include: alkali metal hydroxides such as sodium hydroxide and potassium hydroxide; alkali metal carbonates such as sodium carbonate and potassium carbonate; and phosphorus compounds such as triphenylphosphine. One of these basic catalysts can be used singly, or two or more thereof can be used in combination.

The amount of the basic catalyst used can be appropriately selected depending on its type, a target reaction rate, and the like. In the case of using, for example, an alkali metal hydroxide as the basic catalyst, the amount of the basic catalyst used is preferably 0.01 mol or more and 0.5 mol or less, more preferably 0.01 mol or more and 0.1 mol or less, based on 1 mol of a phenolic hydroxy group of the polyvalent hydroxynaphthalene compound.

The dehydration condensation reaction can be performed in the absence of a solvent or in the presence of a solvent depending on the polyvalent hydroxynaphthalene compound used. The reaction in the absence of a solvent eliminates the need of a solvent collection step and the like. The reaction in the presence of a solvent easily forms a homogeneous reaction liquid. Therefore, the reaction tends to be likely to progress stably.

Examples of the solvent for use in the dehydration condensation reaction include: alcohols such as benzyl alcohol, cyclohexanol, and amyl alcohol; ethylene glycols such as ethylene glycol, diethylene glycol, triethylene glycol, and polyethylene glycol; mono- or di-ethers of ethylene glycol or diethylene glycol, such as ethylene glycol monomethyl ether, ethylene glycol monoethyl ether, ethylene glycol monopropyl ether, diethylene glycol monomethyl ether, diethylene glycol monoethyl ether, diethylene glycol monopropyl ether, diethylene glycol monobutyl ether, ethylene glycol dimethyl ether, ethylene glycol diethyl ether, ethylene glycol dipropyl ether, diethylene glycol dimethyl ether, diethylene glycol diethyl ether, and diethylene glycol dipropyl ether; and chlorobenzene and nitrobenzene. One of these solvents can be used singly, or two or more thereof can be used in combination. Use of such a solvent prevents the salt deposition of the polyvalent hydroxynaphthalene compound during dehydration condensation reaction and can stably produce the phenolic resin having a polynaphthylene ether structure.

The reaction temperature of the dehydration condensation reaction is preferably 100°C or higher and 300°C or lower, more preferably 150°C or higher and 250°C or lower. The reaction time is also preferably in a range that can maintain the reaction temperature conditions described above, and is usually 1 minute or longer and 10 hours or shorter. In the dehydration condensation reaction, it is preferred to distill off water produced during reaction in association with the dehydration condensation reaction to the outside of the system using a fractional distillation tube or the like, from the viewpoint of more improving productivity by rapid progression of the reaction.

After the completion of dehydration condensation reaction, the product is directly solidified to isolate the phenolic resin having a polynaphthylene ether structure, or the catalyst in the product is removed by neutralization treatment, washing treatment with water, or decomposition, and the phenolic resin having a polynaphthylene ether structure can be separated by a general operation such as extraction or distillation. The neutralization treatment or the washing treatment with water can be performed in accordance with an ordinary method. For example, an acidic substance can be used, such as hydrochloric acid, oxalic acid, acetic acid, primary sodium phosphate, or carbon dioxide.

The phenolic resin having a polynaphthylene ether structure thus obtained can be used directly for various purposes; the content of the polyvalent hydroxynaphthalene compound which is an unreacted product may be reduced, if necessary, by a fractionation operation such as distillation, column treatment, or alkaline aqueous solution extraction; or each product may be isolated into a single component.

### Compound represented by formula (27)

Next, the compound represented by the following formula (27) will be described.

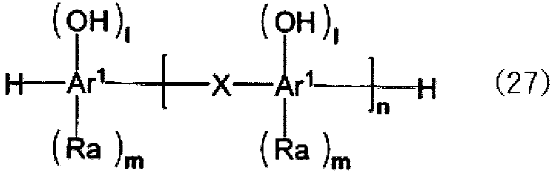

In the formula (27), each Ar¹ independently represents a phenylene group, a naphthylene group, or a biphenylene group, each Ra independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms, I represents the number of hydroxy groups bonded to Ar¹ and each independently represents an integer of 1 or larger and 3 or smaller, m represents the number of Ra bonded to Ar¹ and each independently represents a number determined by subtracting (a + 2) from the number of substitutable substituents in Ar¹, i.e., an integer of 4 - I when Ar¹ is a phenylene group, an integer of 6 - I when Ar¹ is a naphthylene group, and an integer of 8 - I when Ar¹ is a biphenylene group, n is an integer of 0 or larger and 50 or smaller, and each X independently represents a single bond, a divalent organic group having 1 or more and 50 or less carbon atoms in which a hydrogen atom is optionally replaced with a heteroatom, a divalent organic group having 1 or more and 10 or less nitrogen atoms, a carbonyl group, a carboxy group, a carbonyl dioxide group, a sulfonyl group, a divalent sulfur atom, or a divalent oxygen atom, on the proviso that in the formula (27), when n is 0, I is an integer of 2 or larger and 3 or smaller. Each group in the formula (27) may have a substituent.

Each Ar¹ independently represents a phenylene group, a naphthylene group, or a biphenylene group in which a hydrogen atom at an arbitrary position is replaced with a Ra group and a hydroxy group. Each of these groups may have a substituent.

In the formula (27), examples of Ra and X can include the same as those listed in Ra and X, respectively, in the formula (1). For the numbers of repeats, i.e., l, m, and n, see a, b, and c, respectively, in the formula (1).

X in the formula (27) is preferably selected from the group consisting of a divalent organic group having 1 or more and 50 or less carbon atoms, the divalent organic group being represented by the following formula (28), and divalent groups represented by the following formulas (28a), (28b), (28c), (28d), (28e), (28f), (28g), (28h), (28i), and (28j):

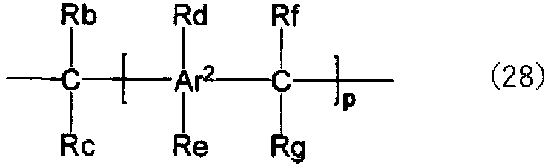

In the formula (28), each Ar² independently represents a phenylene group, a naphthylene group, or a biphenylene group, Rb, Rc, Rf, and Rg each independently represent a hydrogen atom, an alkyl group having 1 or more and 6 or less carbon atoms, or an aryl group having 6 or more and 12 or less carbon atoms, Rd and Re each independently represent a hydrogen atom, an alkyl group having 1 or more and 6 or less carbon atoms, an aryl group having 6 or more and 12 or less carbon atoms, an alkoxy group having 1 or more and 4 or less carbon atoms, or a hydroxy group, and p represents an integer of 0 or larger and 5 or smaller. Each group in the formula (28) may have a substituent.

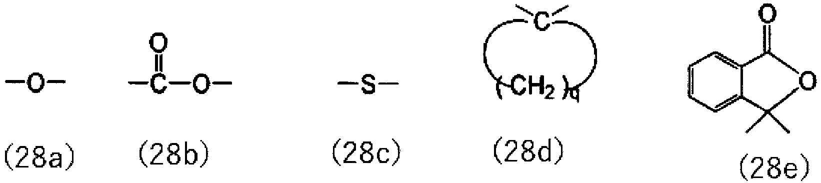

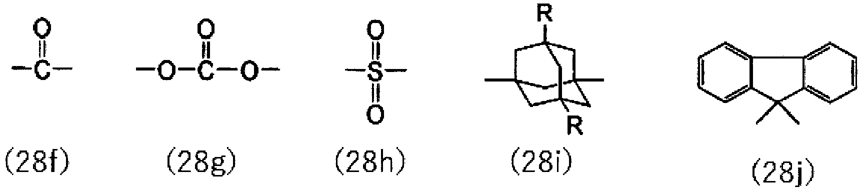

In the formula (28d), q represents an integer of 4 or larger and 7 or smaller. In the formula (28i), each R independently represents a hydrogen atom or an alkyl group having 1 or more and 6 or less carbon atoms.

Each Ar² in the formula (28) independently represents a phenylene group, a naphthylene group, or a biphenylene group. Examples of Ar² include a 1,4-phenylene group, a 1,3-phenylene group, a 2,6-naphthylene group, a 1,5-naphthylene group, a 1,6-naphthylene group, a 1,8-naphthylene group, a 1,3-naphthylene group, a 1,4-naphthylene group, a 4,4'-biphenylene group, a 2,4'-biphenylene group, a 2,2'-biphenylene group, a 2,3'-biphenylene group, a 3,3'-biphenylene group, and a 3,4'-biphenylene group.

Examples of Rb, Rc, Rd, Re, Rf, and Rg in the formula (28) can include the same as those listed in Rc, Rd, Re, Rf, Rg, and Rh, respectively, in the formula (3).

### (27a) 1-Naphthol aralkyl resin

Examples of the 1-naphthol aralkyl resin include resins having a structure where a naphthalene ring having a hydroxy group is bonded to a benzene ring via an alkyl group. Examples of such a 1-naphthol aralkyl resin include compounds of the formula (27) wherein Ar¹ is a naphthylene group, X is represented by the formula (28), and Ar² is a phenylene group. The 1-naphthol aralkyl resin is preferably a compound represented by the formula (29) given below. In the compound represented by the formula (29), two methylene groups bonded to the benzene ring can be bonded to an ortho, meta, or para position. Among them, two methylene groups bonded to the benzene ring are preferably bonded to a meta and/or para position of the benzene ring. Use of such a 1-naphthol aralkyl resin tends to enable the cyanate ester compound to be more preferably produced which has the content of the compound having a triazine group within the range described above, facilitates controlling polymerization reaction at the time of cured product production, and is suitably used in the production of a cured product having desired characteristics.

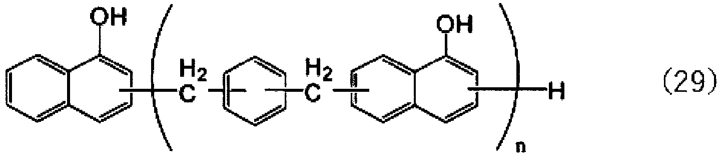

In the formula (29), n is an integer of 1 or larger and 50 or smaller, preferably an integer of 1 or larger and 10 or smaller.

The 1-naphthol aralkyl resin can be obtained, for example, by reacting a bishalogenomethyl compound as represented by Ar²-(CH₂Y)₂ with a naphthol compound in the presence of an acidic catalyst or in the absence of a catalyst, or reacting a bis(alkoxymethyl) compound as represented by Ar²-(CH₂OR)₂ or a bis(hydroxymethyl) compound as represented by Ar²-(CH₂OH)₂ with a naphthol compound in the presence of an acidic catalyst, in accordance with a known method. In this context, Y is a halogen atom. R is an alkyl group. Ar² is as defined in the formula (28).

A commercially available product may be used as the 1-naphthol aralkyl resin. Examples of the commercially available product include SN4 Series Phenolic Resin (trade name, manufactured by NIPPON STEEL Chemical & Material Co., Ltd.).

### (27b) Phenolic resin having adamantane structure

Examples of the phenolic resin having an adamantane structure include resins having a structure where an aromatic ring having a hydroxy group is bonded to an adamantyl group. Examples of such a phenolic resin include compounds of the formula (27) wherein X is a group represented by the formula (28i). Specific examples thereof include compounds represented by the formula (30) given below. Use of such a compound tends to enable the cyanate ester compound to be more preferably produced which has the content of the compound having a triazine ring within the range described above, facilitates controlling polymerization reaction at the time of cured product production, and is suitably used in the production of a cured product having desired characteristics.

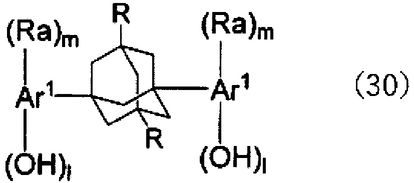

In the formula (30), each Ar¹ independently represents a phenylene group, a naphthylene group, or a biphenylene group, R is the same as R in the formula (28i), Ra is the same as Ra in the formula (27), I represents the number of hydroxy groups bonded to Ar¹ and is an integer of 1 or larger and 3 or smaller, m represents the number of Ra bonded to Ar¹ and is an integer of 5 - I when Ar¹ is a phenylene group, an integer of 7 - I when Ar¹ is a naphthylene group, and an integer of 9 - I when Ar¹ is a biphenylene group.

Examples of such a phenolic resin having an adamantane structure include 1,3-bis(4-hydroxyphenyl)adamantane, 1,3-bis(4-hydroxyphenyl)-5,7-dimethyladamantane, 1,3-bis(4-hydroxyphenyl)-5-methyladamantane, 1,3-bis(4-hydroxyphenyl)-5-ethyladamantane, 1,3-bis(4-hydroxyphenyl)-5-propyladamantane, 1,3-bis(4-hydroxyphenyl)-5-isopropyladamantane, 1,3-bis(4-hydroxyphenyl)-5-t-butyladamantane, 1,3-bis(4-hydroxyphenyl)-5,7-dimethyladamantane, 1,3-bis(4-hydroxyphenyl)-5-methyl-7-ethyladamantane, 1,3-bis(4-hydroxyphenyl)-5-methyl-7-propyladamantane, 1,3-bis(4-hydroxyphenyl)-5-ethyl-7-propyladamantane, 1,3-bis(4-hydroxyphenyl)-5,7-dipropyladamantane, 1,3-bis(4-hydroxyphenyl)-5-methyl-7-isopropyladamantane, 1,3-bis(4-hydroxyphenyl)-5-ethyl-7-isopropyladamantane, 1,3-bis(4-hydroxyphenyl)-5-propyl-7-isopropyladamantane, 1,3-bis(4-hydroxyphenyl)-5,7-diisopropyladamantane, 1,3-bis(4-hydroxyphenyl)-5-methyl-7-t-butyladamantane, 1,3-bis(4-hydroxyphenyl)-5-ethyl-7-t-butyladamantane, 1,3-bis(4-hydroxyphenyl)-5-propyl-7-t-butyladamantane, 1,3-bis(4-hydroxyphenyl)-5-isopropyl-7-t-butyladamantane, 1,3-bis(4-hydroxyphenyl)-5,7-di-t-butyladamantane, 1,3-bis(3-methyl-4-hydroxyphenyl)-5-methyladamantane, 1,3-bis(3-methyl-4-hydroxyphenyl)-5-ethyladamantane, 1,3-bis(3-methyl-4-hydroxyphenyl)-5-propyladamantane, 1,3-bis(3-methyl-4-hydroxyphenyl)-5-isopropyladamantane, 1,3-bis(3-methyl-4-hydroxyphenyl)-5-t-butyladamantane, 1,3-bis(3-methyl-4-hydroxyphenyl)-5,7-dimethyladamantane, 1,3-bis(3-methyl-4-hydroxyphenyl)-5-methyl-7-ethyladamantane, 1,3-bis(3-methyl-4-hydroxyphenyl)-5,7-diethyladamantane, 1,3-bis(3-methyl-4-hydroxyphenyl)-5-methyl-7-propyladamantane, 1,3-bis(3-methyl-4-hydroxyphenyl)-5-ethyl-7-propyladamantane, 1,3-bis(3-methyl-4-hydroxyphenyl)-5,7-dipropyladamantane, 1,3-bis(3-methyl-4-hydroxyphenyl)-5-methyl-7-isopropyladamantane, 1,3-bis(3-methyl-4-hydroxyphenyl)-5-ethyl-7-isopropyladamantane, 1,3-bis(3-methyl-4-hydroxyphenyl)-5-propyl-7-isopropyladamantane, 1,3-bis(3-methyl-4-hydroxyphenyl)-5,7-diisopropyladamantane, 1,3-bis(3-methyl-4-hydroxyphenyl)-5-methyl-7-t-butyladamantane, 1,3-bis(3-methyl-4-hydroxyphenyl)-5-ethyl-7-t-butyladamantane, 1,3-bis(3-methyl-4-hydroxyphenyl)-5-propyl-7-t-butyladamantane, 1,3-bis(3-methyl-4-hydroxyphenyl)-5-isopropyl-7-t-butyladamantane, 1,3-bis(3-methyl-4-hydroxyphenyl)-5,7-di-t-butyladamantane, 1,3-bis(3,5-dimethyl-4-hydroxyphenyl)-5-methyladamantane, 1,3-bis(3,5-dimethyl-4-hydroxyphenyl)-5-ethyladamantane, 1,3-bis(3,5-dimethyl-4-hydroxyphenyl)-5-propyladamantane, 1,3-bis(3,5-dimethyl-4-hydroxyphenyl)-5-isopropyladamantane, 1,3-bis(3,5-dimethyl-4-hydroxyphenyl)-5-t-butyladamantane, 1,3-bis(3,5-dimethyl-4-hydroxyphenyl)-5,7-dimethyladamantane, 1,3-bis(3,5-dimethyl-4-hydroxyphenyl)-5-methyl-7-ethyladamantane, 1,3-bis(3,5-dimethyl-4-hydroxyphenyl)-5,7-diethyladamantane, 1,3-bis(3,5-dimethyl-4-hydroxyphenyl)-5-methyl-7-propyladamantane, 1,3-bis(3,5-dimethyl-4-hydroxyphenyl)-5-ethyl-7-propyladamantane, 1,3-bis(3,5-dimethyl-4-hydroxyphenyl)-5,7-dipropyladamantane, 1,3-bis(3,5-dimethyl-4-hydroxyphenyl)-5-methyl-7-isopropyladamantane, 1,3-bis(3,5-dimethyl-4-hydroxyphenyl)-5-ethyl-7-isopropyladamantane, 1,3-bis(3,5-dimethyl-4-hydroxyphenyl)-5-propyl-7-isopropyladamantane, 1,3-bis(3,5-dimethyl-4-hydroxyphenyl)-5,7-diisopropyladamantane, 1,3-bis(3,5-dimethyl-4-hydroxyphenyl)-5-methyl-7-t-butyladamantane, 1,3-bis(3,5-dimethyl-4-hydroxyphenyl)-5-ethyl-7-t-butyladamantane, 1,3-bis(3,5-dimethyl-4-hydroxyphenyl)-5-propyl-7-t-butyladamantane, 1,3-bis(3,5-dimethyl-4-hydroxyphenyl)-5-isopropyl-7-t-butyladamantane, 1,3-bis(3,5-dimethyl-4-hydroxyphenyl)-5,7-di-t-butyladamantane, 1,3-bis(3-phenyl-4-hydroxyphenyl)-5-methyladamantane, 1,3-bis(3-phenyl-4-hydroxyphenyl)-5-ethyladamantane, 1,3-bis(3-phenyl-4-hydroxyphenyl)-5-propyladamantane, 1,3-bis(3-phenyl-4-hydroxyphenyl)-5-isopropyladamantane, 1,3-bis(3-phenyl-4-hydroxyphenyl)-5-t-butyladamantane, 1,3-bis(3-phenyl-4-hydroxyphenyl)-5,7-dimethyladamantane, 1,3-bis(3-phenyl-4-hydroxyphenyl)-5-methyl-7-ethyladamantane,
1,3-bis(3-phenyl-4-hydroxyphenyl)-5,7-diethyladamantane, 1,3-bis(3-phenyl-4-hydroxyphenyl)-5-methyl-7-propyladamantane, 1,3-bis(3-phenyl-4-hydroxyphenyl)-5-ethyl-7-propyladamantane, 1,3-bis(3-phenyl-4-hydroxyphenyl)-5,7-dipropyladamantane, 1,3-bis(3-phenyl-4-hydroxyphenyl)-5-methyl-7-isopropyladamantane, 1,3-bis(3-phenyl-4-hydroxyphenyl)-5-ethyl-7-isopropyladamantane, 1,3-bis(3-phenyl-4-hydroxyphenyl)-5-propyl-7-isopropyladamantane, 1,3-bis(3-phenyl-4-hydroxyphenyl)-5,7-diisopropyladamantane, 1,3-bis(3-phenyl-4-hydroxyphenyl)-5-methyl-7-t-butyladamantane, 1,3-bis(3-phenyl-4-hydroxyphenyl)-5-ethyl-7-t-butyladamantane, 1,3-bis(3-phenyl-4-hydroxyphenyl)-5-propyl-7-t-butyladamantane, 1,3-bis(3-phenyl-4-hydroxyphenyl)-5-isopropyl-7-t-butyladamantane, 1,3-bis(3-phenyl-4-hydroxyphenyl)-5,7-di-t-butyladamantane, 1,3-bis(3-cyclohexyl-4-hydroxyphenyl)-5-methyladamantane, 1,3-bis(3-cyclohexyl-4-hydroxyphenyl)-5-ethyladamantane, 1,3-bis(3-cyclohexyl-4-hydroxyphenyl)-5-propyladamantane, 1,3-bis(3-cyclohexyl-4-hydroxyphenyl)-5-isopropyladamantane, 1,3-bis(3-cyclohexyl-4-hydroxyphenyl)-5-t-butyladamantane, 1,3-bis(3-cyclohexyl-4-hydroxyphenyl)-5,7-dimethyladamantane, 1,3-bis(3-cyclohexyl-4-hydroxyphenyl)-5-methyl-7-ethyladamantane, 1,3-bis(3-cyclohexyl-4-hydroxyphenyl)-5,7-diethyladamantane, 1,3-bis(3-cyclohexyl-4-hydroxyphenyl)-5-methyl-7-propyladamantane, 1,3-bis(3-cyclohexyl-4-hydroxyphenyl)-5-ethyl-7-propyladamantane, 1,3-bis(3-cyclohexyl-4-hydroxyphenyl)-5,7-dipropyladamantane, 1,3-bis(3-cyclohexyl-4-hydroxyphenyl)-5-methyl-7-isopropyladamantane, 1,3-bis(3-cyclohexyl-4-hydroxyphenyl)-5-ethyl-7-isopropyladamantane, 1,3-bis(3-cyclohexyl-4-hydroxyphenyl)-5-propyl-7-isopropyladamantane, 1,3-bis(3-cyclohexyl-4-hydroxyphenyl)-5,7-diisopropyladamantane, 1,3-bis(3-cyclohexyl-4-hydroxyphenyl)-5-methyl-7-t-butyladamantane, 1,3-bis(3-cyclohexyl-4-hydroxyphenyl)-5-ethyl-7-t-butyladamantane, 1,3-bis(3-cyclohexyl-4-hydroxyphenyl)-5-propyl-7-t-butyladamantane, 1,3-bis(3-cyclohexyl-4-hydroxyphenyl)-5-isopropyl-7-t-butyladamantane, 1,3-bis(3-cyclohexyl-4-hydroxyphenyl)-5,7-di-t-butyladamantane, 1,3-bis(4-methyl-2-hydroxyphenyl)-5-methyladamantane, 1,3-bis(4-methyl-2-hydroxyphenyl)-5-ethyladamantane, 1,3-bis(4-methyl-2-hydroxyphenyl)-5-propyladamantane, 1,3-bis(4-methyl-2-hydroxyphenyl)-5-isopropyladamantane, 1,3-bis(4-methyl-2-hydroxyphenyl)-5-t-butyladamantane, 1,3-bis(4-methyl-2-hydroxyphenyl)-5,7-dimethyladamantane, 1,3-bis(4-methyl-2-hydroxyphenyl)-5-methyl-7-ethyladamantane, 1,3-bis(4-methyl-2-hydroxyphenyl)-5,7-diethyladamantane, 1,3-bis(4-methyl-2-hydroxyphenyl)-5-methyl-7-propyladamantane, 1,3-bis(4-methyl-2-hydroxyphenyl)-5-ethyl-7-propyladamantane, 1,3-bis(4-methyl-2-hydroxyphenyl)-5,7-dipropyladamantane, 1,3-bis(4-methyl-2-hydroxyphenyl)-5-methyl-7-isopropyladamantane, 1,3-bis(4-methyl-2-hydroxyphenyl)-5-ethyl-7-isopropyladamantane, 1,3-bis(4-methyl-2-hydroxyphenyl)-5-propyl-7-isopropyladamantane, 1,3-bis(4-methyl-2-hydroxyphenyl)-5,7-diisopropyladamantane, 1,3-bis(4-methyl-2-hydroxyphenyl)-5-methyl-7-t-butyladamantane, 1,3-bis(4-methyl-2-hydroxyphenyl)-5-ethyl-7-t-butyladamantane, 1,3-bis(4-methyl-2-hydroxyphenyl)-5-propyl-7-t-butyladamantane, 1,3-bis(4-methyl-2-hydroxyphenyl)-5-isopropyl-7-t-butyladamantane, 1,3-bis(4-methyl-2-hydroxyphenyl)-5,7-di-t-butyladamantane, and 1,3-bis(2,4-dihydroxyphenyl)-adamantane.

### (27c) Other hydroxy-substituted aromatic compounds

Examples of other hydroxy-substituted aromatic compounds other than the 1-naphthol aralkyl resin and the phenolic resin having an adamantane structure as the compound represented by the formula (27) include 2,3-,2,4-,2,5-,2,6-,3,4- or 3,5-xylenol, catechol, resorcinol, hydroquinone, 2-tert-butylhydroquinone, 2,4-dimethylhydroquinone, tetramethylhydroquinone, 2,4,6-trimethylresorcinol, 3,5-dihydroxytoluene, 2,2'-dihydroxy-1,1'-binaphthyl, 1,3-,1,4-,1,5-,1,6-,1,7-,2,3-,2,6- or 2,7-dihydroxynaphthalene, 2,2'- or 4,4'-dihydroxybiphenyl, 4,4'-dihydroxyoctafluorobiphenyl, 2,2'- or 4,4'-dihydroxydiphenylmethane, bis(4-hydroxy-3,5-dimethylphenyl)methane, 1,1-bis(4-hydroxyphenyl)ethane, 1,1-bis(4-hydroxyphenyl)propane, 2,2-bis(4-hydroxyphenyl)propane, 2,2-bis(4-hydroxy-3-methylphenyl)propane, 2,2-bis(2-hydroxy-5-biphenylyl)propane, 2,2-bis(4-hydroxyphenyl)hexafluoropropane, 2,2-bis(4-hydroxy-3,5-dimethylphenyl)propane, 1,1-bis(4-hydroxyphenyl)butane, 1,1-bis(4-hydroxyphenyl)isobutane, 1,1-bis(4-hydroxyphenyl)pentane, 1,1-bis(4-hydroxyphenyl)-3-methylbutane, 1,1-bis(4-hydroxyphenyl)-2-methylbutane, 1,1-bis(4-hydroxyphenyl)-2,2-dimethylpropane, 2,2-bis(4-hydroxyphenyl)butane, 2,2-bis(4-hydroxyphenyl)pentane, 2,2-bis(4-hydroxyphenyl)hexane, 2,2-bis(4-hydroxyphenyl)-3-methylbutane, 2,2-bis(4-hydroxyphenyl)-4-methylpentane, 2,2-bis(4-hydroxyphenyl)-3-methylpentane, 2,2-bis(4-hydroxyphenyl)-3,3-dimethylbutane, 3,3-bis(4-hydroxyphenyl)hexane, 3,3-bis(4-hydroxyphenyl)heptane, 3,3-bis(4-hydroxyphenyl)octane, 3,3-bis(4-hydroxyphenyl)-2-methylpentane, 3,3-bis(4-hydroxyphenyl)-2-methylhexane, 3,3-bis(4-hydroxyphenyl)-2,2-dimethylpentane, 4,4-bis(4-hydroxyphenyl)-3-methylheptane, 3,3-bis(4-hydroxyphenyl)-2-methylheptane, 3,3-bis(4-hydroxyphenyl)-2,2-dimethylhexane, 3,3-bis(4-hydroxyphenyl)-2,4-dimethylhexane, 3,3-bis(4-hydroxyphenyl)-2,2,4-trimethylpentane, 2,2-bis(4-hydroxyphenyl)-1,1,1,3,3,3-hexafluoropropane, bis(4-hydroxyphenyl)phenylmethane, 1,1-bis(4-hydroxyphenyl)-1-phenylethane, bis(4-hydroxyphenyl)biphenylmethane, 1,1-bis(4-hydroxyphenyl)cyclopentane, 1,1-bis(4-hydroxyphenyl)cyclohexane, 2,2-bis(4-hydroxy-3-isopropylphenyl)propane, 1,1-bis(3-cyclohexyl-4-hydroxyphenyl)cyclohexane, bis(4-hydroxyphenyl)diphenylmethane, bis(4-hydroxyphenyl)-2,2-dichloroethylene, 1,3-bis[2-(4-hydroxyphenyl)-2-propyl]benzene, 1,4-bis[2-(4-hydroxyphenyl)-2-propyl]benzene, 1,1-bis(4-hydroxyphenyl)-3,3,5-trimethylcyclohexane, 4-[bis(4-hydroxyphenyl)methyl]biphenyl, 4,4'-dihydroxybenzophenone, 1,3-bis(4-hydroxyphenyl)-2-propen-1-one, bis(4-hydroxyphenyl) ether, bis(4-hydroxyphenyl) sulfide, bis(4-hydroxyphenyl)sulfone, 4-hydroxybenzoic acid-4-hydroxyphenyl ester (4-hydroxyphenyl-4-hydroxybenzoate), bis(4-hydroxyphenyl) carbonate, phenolphthalein, o-cresolphthalein, 9,9-bis(4-hydroxyphenyl)fluorene, 9,9-bis(4-hydroxy-3-methylphenyl)fluorene, 9,9-bis(2-hydroxy-5-biphenylyl)fluorene, tris(4-hydroxyphenyl)methane, 1,1,1-tris(4-hydroxyphenyl)ethane, 1,1,3-tris(4-hydroxyphenyl)propane, α,α,α'-tris(4-hydroxyphenyl)-1-ethyl-4-isopropylbenzene, 1,1,2,2-tetrakis(4-hydroxyphenyl)ethane, 4,4',4",4"'-methanetetrayltetrakisphenol, 2,4,6-tris(N-methyl-4-hydroxyanilino)-1,3,5-triazine, 2,4-bis(N-methyl-4-hydroxyanilino)-6-(N-methylanilino)-1,3,5-triazine, bis(N-4-hydroxy-2-methylphenyl)-4,4'-oxydiphthalimide, bis(N-3-hydroxy-4-methylphenyl)-4,4'-oxydiphthalimide, bis(N-4-hydroxyphenyl)-4,4'-oxydiphthalimide, bis(N-4-hydroxy-2-methylphenyl)-4,4'-(hexafluoroisopropylidene)diphthalimide, tris(3,5-dimethyl-4-hydroxybenzyl) isocyanurate, 2-phenyl-3,3-bis(4-hydroxyphenyl)phthalimidine, 2-(4-methylphenyl)-3,3-bis(4-hydroxyphenyl)phthalimidine, 2-phenyl-3,3-bis(4-hydroxy-3-methylphenyl)phthalimidine, 1-methyl-3,3-bis(4-hydroxyphenyl)indolin-2-one, 2-phenyl-3,3-bis(4-hydroxyphenyl)indolin-2-one, phenol novolac resin, cresol novolac resin, phenol aralkyl resin, cresol aralkyl resin, biphenyl aralkyl resin, phenol-modified xylene formaldehyde resin, and phenol-modified dicyclopentadiene resin.

Examples of the phenol novolac resin and the cresol novolac resin include resins obtained by reacting phenol, alkyl-substituted phenol, or halogen-substituted phenol with a formaldehyde compound such as formalin or paraformaldehyde in an acidic solution in accordance with a known method.

Examples of the phenol aralkyl resin, the cresol aralkyl resin, and the biphenyl aralkyl resin include resins obtained by reacting a bishalogenomethyl compound as represented by Ar²-(CH₂Y)₂ with a phenolic compound in the presence of an acidic catalyst or in the absence of a catalyst, and resins obtained by reacting a bis(alkoxymethyl) compound as represented by Ar²-(CH₂OR)₂ or a bis(hydroxymethyl) compound as represented by Ar²-(CH₂OH)₂ with a phenolic compound in the presence of an acidic catalyst, in accordance with a known method. In this context, Y is a halogen atom. R is an alkyl group. Ar² is as defined in the formula (28).

Examples of the phenol-modified xylene formaldehyde resin include resins obtained by reacting xylene formaldehyde resin with a phenolic compound in the presence of an acidic catalyst in accordance with a known method.

### (Basic compound)

In the cyanation step, the basic compounds 1 and 2 is used as a desalting agent in the cyanation of the hydroxy-substituted aromatic compound with the cyanogen halide. Examples of such a basic compound include organic bases and inorganic bases. The basic compound may be used in a solid state or used in a solution state.

The organic base is preferably, tertiary amine, for example, trimethylamine, triethylamine, tri-n-butylamine, triamylamine, diisopropylethylamine, diethyl-n-butylamine, methyl-di-n-butylamine, methylethyl-n-butylamine, dodecyldimethylamine, tribenzylamine, triethanolamine, N,N-dimethylaniline, N,N-diethylaniline, diphenylmethylamine, pyridine, diethylcyclohexylamine, tricyclohexylamine, 1,4-diazabicyclo[2.2.2]octane, 1,8-diazabicyclo[5.4.0]-7-undecene, and 1,5-diazabicyclo[4.3.0]-5-nonene. Among them, the basic compound is more preferably one or more members selected from the group consisting of trimethylamine, triethylamine, tri-n-butylamine, and diisopropylethylamine, further preferably triethylamine. Use of such a basic compound tends to enable the cyanate ester compound to be more preferably produced at a high yield, which has the content of the compound having a triazine ring within the range described above, facilitates controlling polymerization reaction at the time of cured product production, and is suitably used in the production of a cured product having desired characteristics.

The inorganic base is preferably hydroxide of an alkali metal, for example, sodium hydroxide, potassium hydroxide, and lithium hydroxide. Among them, sodium hydroxide is more preferred from the viewpoint of being inexpensively available.

In the cyanation step, the amount (total amount) of the basic compounds 1 and 2 is preferably 0.1 mol or more and 8.0 mol or less, more preferably 1.0 mol or more and 6.0 mol or less, based on 1 mol of a hydroxy group of the hydroxy-substituted aromatic compound. The amount of the basic compound used within the range described above tends to enable the cyanate ester compound to be further preferably produced at a high yield, which has the content of the compound having a triazine ring within the range described above, facilitates controlling polymerization reaction at the time of cured product production, and is suitably used in the production of a cured product having desired characteristics.

In the cyanation step, the basic compound can be dissolved in water or an organic solvent and used as a solution. When the basic compound is an organic base, an organic solvent is preferably used. When the basic compound is an inorganic base, water is preferably used.

When the solution containing the basic compound contains the hydroxy-substituted aromatic compound, the content of the solvent in the solution containing the basic compound is preferably 0.10 parts by mass or more and 100 parts by mass or less, more preferably 0.10 parts by mass or more and 80 parts by mass or less, based on 1 part by mass of the hydroxy-substituted aromatic compound. This solution containing the basic compound is, for example, the solution 1 in the starting material solution preparation step.

When the solution containing the basic compound contains no hydroxy-substituted aromatic compound, the content of the solvent in the solution containing the basic compound is preferably 0.10 parts by mass or more and 100 parts by mass or less based on 1 part by mass of the basic compound. This solution containing the basic compound is, for example, the solution 3 in the cyanation step.

For examples of the organic solvent and the water, see the above description.

The organic solvent is preferably immiscible with water and inert to cyanation reaction. Use of the organic solvent immiscible with water can collect an organic solvent layer containing the cyanate ester compound from the reaction liquid which is the mixed system of the organic solvent and the water at the end point of the cyanation step, and can separate the cyanate ester compound.

### [Resin composition]

The resin composition of the present embodiment contains the cyanate ester compound of the present embodiment.

One cyanate ester compound can be used singly, or two or more cyanate ester compounds can be used in combination.

The content of the cyanate ester compound is preferably 1.0 part by mass or more and 100 parts by mass or less based on 100 parts by mass of the resin solid content in the resin composition. The content of the cyanate ester compound falls within the range described above, whereby the resulting resin composition tends to have better heat resistance, low dielectricity, and low dissipation factor, etc.

The resin composition may optionally further contain one or more members selected from the group consisting of a cyanate ester compound other than the cyanate ester compound of the present embodiment (hereinafter, also referred to as an "additional cyanate ester compound"), an epoxy resin, an oxetane resin, a maleimide compound, a phenolic resin, a benzoxazine compound, a compound having a polymerizable unsaturated group, and a filler. The resin composition thus configured can produce a cured product excellent in flame retardancy, low water absorbability, moisture-absorbing heat resistance, heat resistance, low thermal expansibility, low dielectricity, and low dissipation factor, etc.

### (Epoxy resin)

The resin composition may contain an epoxy resin. The resin composition containing the epoxy resin tends to have better adhesion, moisture-absorbing heat resistance, and flexibility, etc.

For example, a generally known compound can be used as the epoxy resin as long as the compound has two or more epoxy groups in one molecule. Specific examples thereof include bisphenol A-type epoxy resin, bisphenol E-type epoxy resin, bisphenol F-type epoxy resin, bisphenol S-type epoxy resin, bisphenol A novolac-type epoxy resin, biphenyl-type epoxy resin, phenol novolac-type epoxy resin, cresol novolac-type epoxy resin, xylene novolac-type epoxy resin, polyfunctional phenol-type epoxy resin, naphthalene-type epoxy resin, naphthalene backbone-modified novolac-type epoxy resin, naphthylene ether-type epoxy resin, phenol aralkyl-type epoxy resin, anthracene-type epoxy resin, trifunctional phenol-type epoxy resin, tetrafunctional phenol-type epoxy resin, triglycidyl isocyanurate, glycidyl ester-type epoxy resin, alicyclic epoxy resin, dicyclopentadiene novolac-type epoxy resin, biphenyl novolac-type epoxy resin, phenol aralkyl novolac-type epoxy resin, naphthol aralkyl novolac-type epoxy resin, aralkyl novolac-type epoxy resin, biphenyl aralkyl-type epoxy resin, naphthol aralkyl-type epoxy resin, dicyclopentadiene-type epoxy resin, polyol-type epoxy resin, phosphorus-containing epoxy resin, glycidylamine, compounds having an epoxidized double bond such as butadiene, compounds obtained through the reaction of hydroxy group-containing silicone resins with epichlorohydrin, and halides thereof. One of these epoxy resins can be used singly, or two or more thereof can be used in combination.

Among them, the epoxy resin is preferably one or more members selected from the group consisting of biphenyl aralkyl-type epoxy resin, naphthylene ether-type epoxy resin, polyfunctional phenol-type epoxy resin, and naphthalene-type epoxy resin. The resin composition containing such an epoxy resin tends to more improve the flame retardancy and heat resistance of the resulting cured product.

The content of the epoxy resin is preferably 0.0 parts by mass or more and 99 parts by mass or less, more preferably 1.0 part by mass or more and 90 parts by mass or less, based on 100 parts by mass of the resin solid content in the resin composition. The content of the epoxy resin falls within the range described above, whereby the resulting resin composition tends to have better adhesion and flexibility, etc.

### (Oxetane resin)

The resin composition may contain an oxetane resin. The resin composition containing the oxetane resin tends to have better adhesion and flexibility, etc.

A generally known compound can be used as the oxetane resin. Specific examples thereof include oxetane, alkyloxetane such as 2-methyloxetane, 2,2-dimethyloxetane, 3-methyloxetane, and 3,3-dimethyloxetane, 3-methyl-3-methoxymethyloxetane, 3,3'-di(trifluoromethyl)perfluorooxetane, 2-chloromethyloxetane, 3,3-bis(chloromethyl)oxetane, biphenyl-type oxetane, OXT-101 (trade name, manufactured by Toagosei Co., Ltd.), and OXT-121 (trade name, manufactured by Toagosei Co., Ltd.). One of these oxetane resins can be used singly, or two or more thereof can be used in combination.

The content of the oxetane resin is preferably 0.0 parts by mass or more and 99 parts by mass or less, more preferably 1.0 part by mass or more and 90 parts by mass or less, based on 100 parts by mass of the resin solid content in the resin composition. The content of the oxetane resin falls within the range described above, whereby the resulting resin composition tends to have better close contact and flexibility, etc.

### (Maleimide compound)

The resin composition may contain a maleimide compound. The resin composition containing the maleimide compound tends to have better heat resistance, moisture-absorbing heat resistance, and toughness, etc.

A generally known compound can be used as the maleimide compound as long as the compound has one or more maleimide groups in one molecule. Specific examples thereof include 4,4'-diphenylmethane bismaleimide, phenylmethane maleimide, m-phenylene bismaleimide, 2,2-bis(4-(4-maleimidophenoxy)-phenyl)propane, 3,3'-dimethyl-5,5'-diethyl-4,4'-diphenylmethane bismaleimide, 4-methyl-1,3-phenylene bismaleimide, 1,6- bismaleimide-(2,2,4-trimethyl)hexane, 4,4'-diphenyl ether bismaleimide, 4,4'-diphenylsulfone bismaleimide, 1,3-bis(3-maleimidophenoxy)benzene, 1,3-bis(4-maleimidophenoxy)benzene, polyphenylmethane maleimide, and prepolymers of these maleimide compounds, and prepolymers of the maleimide compounds and amine compounds. One of these maleimide compounds can be used singly, or two or more thereof can be used in combination.

The content of the maleimide compound is preferably 0.0 parts by mass or more and 99 parts by mass or less, more preferably 1.0 part by mass or more and 90 parts by mass or less, based on 100 parts by mass of the resin solid content in the resin composition. The content of the maleimide compound falls within the range described above, whereby the resulting resin composition tends to have better heat resistance, etc.

### (Phenolic resin)

The resin composition may contain a phenolic resin. The resin composition containing the phenolic resin tends to have better adhesion and flexibility, etc.

A generally known phenolic resin can be used as the phenolic resin as long as the phenolic resin has two or more hydroxy groups in one molecule. Specific examples thereof include bisphenol A-type phenolic resin, bisphenol E-type phenolic resin, bisphenol F-type phenolic resin, bisphenol S-type phenolic resin, phenol novolac resin, bisphenol A novolac-type phenolic resin, aralkyl novolac-type phenolic resin, biphenyl aralkyl-type phenolic resin, cresol novolac-type phenolic resin, polyfunctional phenolic resin, naphthol resin, naphthol novolac resin, polyfunctional naphthol resin, anthracene-type phenolic resin, naphthalene backbone-modified novolac-type phenolic resin, phenol aralkyl-type phenolic resin, naphthol aralkyl-type phenolic resin, dicyclopentadiene-type phenolic resin, biphenyl-type phenolic resin, alicyclic backbone-containing phenolic resin, phosphorus-containing phenolic resin, and hydroxy group-modified silicone resins. One of these phenolic resins can be used singly, or two or more thereof can be used in combination.

The content of the phenolic resin is preferably 0.0 parts by mass or more and 99 parts by mass or less, more preferably 1.0 part by mass or more and 90 parts by mass or less, based on 100 parts by mass of the resin solid content in the resin composition. The content of the phenolic resin falls within the range described above, whereby the resulting resin composition tends to have better adhesion and flexibility, etc.

### (Benzoxazine compound)

The resin composition may contain a benzoxazine compound. The resin composition containing the benzoxazine compound tends to have better flame retardancy, heat resistance, low water absorbability, and low dielectricity, etc.

A generally known compound can be used as the benzoxazine compound as long as the compound has two or more dihydrobenzoxazine rings in one molecule. Specific examples thereof include bisphenol A-type benzoxazine BA-BXZ (trade name, manufactured by Konishi Chemical Ind. Co., Ltd.), bisphenol F-type benzoxazine BF-BXZ (trade name, manufactured by Konishi Chemical Ind. Co., Ltd.), and bisphenol S-type benzoxazine BS-BXZ (trade name, manufactured by Konishi Chemical Ind. Co., Ltd.). One of these benzoxazine compounds can be used singly, or two or more thereof can be used in combination.

The content of the benzoxazine compound is preferably 0.0 parts by mass or more and 99 parts by mass or less, more preferably 1.0 part by mass or more and 90 parts by mass or less, based on 100 parts by mass of the resin solid content in the resin composition. The content of the benzoxazine compound falls within the range described above, whereby the resulting resin composition tends to have better heat resistance, etc.

### (Compound having polymerizable unsaturated group)

The resin composition may contain a compound having a polymerizable unsaturated group. The resin composition containing the compound having a polymerizable unsaturated group tends to have better heat resistance and toughness, etc.

A generally known compound can be used as the compound having a polymerizable unsaturated group. Specific examples thereof include: vinyl compounds such as ethylene, propylene, styrene, divinylbenzene, and divinylbiphenyl; monohydric or polyhydric alcohol (meth)acrylates such as methyl (meth)acrylate, 2-hydroxyethyl (meth)acrylate, 2-hydroxypropyl (meth)acrylate, polypropylene glycol di(meth)acrylate, trimethylolpropane di(meth)acrylate, trimethylolpropane tri(meth)acrylate, pentaerythritol tetra(meth)acrylate, and dipentaerythritol hexa(meth)acrylate; epoxy (meth)acrylates such as bisphenol A-type epoxy (meth)acrylate and bisphenol F-type epoxy (meth)acrylate; and benzocyclobutene resin. One of these compounds having a polymerizable unsaturated group can be used singly, or two or more thereof can be used in combination.

The content of the compound having a polymerizable unsaturated group is preferably 0.0 parts by mass or more and 99 parts by mass or less, more preferably 1.0 part by mass or more and 90 parts by mass or less, based on 100 parts by mass of the resin solid content in the resin composition. The content of the compound having a polymerizable unsaturated group falls within the range described above, whereby the resulting resin composition tends to have better heat resistance and toughness, etc.

### (Filler)

The resin composition may contain a filler. The resin composition containing the filler tends to have better flame retardancy, low thermal expansibility, high heat conductivity, and toughness, etc.

Examples of the filler include inorganic fillers and organic fillers. One of these fillers can be used singly, or two or more thereof can be used in combination.

A generally known filler can be used as the inorganic filler. Specific examples thereof include: silicates such as kaolin, fired kaolin, talc, fired talc, fired clay, unfired clay, mica, E glass, A glass, NE glass, C glass, L glass, D glass, S glass, M glass G20, short glass fiber (including fine glass powders such as E glass, T glass, D glass, S glass, and Q glass), hollow glass, and spherical glass; silicas such as white carbon (wet silica), natural silica, molten silica, synthetic silica, amorphous silica, Aerosil, and hollow silica; oxides such as titanium oxide, alumina, boehmite, zinc oxide, magnesium oxide, and zirconium oxide; carbonates such as calcium carbonate, magnesium carbonate, and hydrotalcite; hydroxides such as aluminum hydroxide, heat-treated products of aluminum hydroxide (which is obtained by heat-treating aluminum hydroxide and removing a portion of **crystallization** water), magnesium hydroxide, and calcium hydroxide; sulfates or sulfites such as barium sulfate, calcium sulfate, and calcium sulfite; borates such as zinc borate, barium metaborate, aluminum borate, calcium borate, and sodium borate; nitrides such as aluminum nitride, boron nitride, boron nitride agglomerates, silicon nitride, and carbon nitride; titanates such as strontium titanate and barium titanate; stannates such as zinc stannate; and molybdenum compounds such as molybdenum oxide and zinc molybdate. One of these inorganic fillers can be used singly, or two or more thereof can be used in combination.

Examples of the organic filler include: rubber powders such as styrene-type powders, butadiene-type powders, and acrylic powders; core-shell-type rubber powders; silicone resin powders; silicone rubber powders; and silicone composite powders. One of these organic fillers can be used singly, or two or more thereof can be used in combination.

The filler may be used in combination with a silane coupling agent or a wetting and dispersing agent. The resin composition may contain one or more members selected from the group consisting of a silane coupling agent and a wetting and dispersing agent.

A compound that is generally used in the surface treatment of inorganic matter or organic matter can be suitably used as the silane coupling agent. Specific examples thereof include: aminosilane compounds such as γ-aminopropyltriethoxysilane and N-β-(aminoethyl)-y-aminopropyltrimethoxysilane; **epoxysilane** compounds such as γ-glycidoxypropyltrimethoxysilane and β-(3,4-epoxycyclohexyl)ethyltrimethoxysilane; vinylsilane compounds such as γ-methacryloxypropyltrimethoxysilane and vinyl-tri(β-methoxyethoxy)silane; cationic silane compounds such as N-β-(N-vinylbenzylaminoethyl)-y-aminopropyltrimethoxysilane hydrochloride; and phenylsilane compounds. One of these silane coupling agents can be used singly, or two or more thereof can be used in combination.

A compound that is generally used for paints can be suitably used as the wetting and dispersing agent. Preferably, a copolymer-based wetting and dispersing agent is used. Specific examples thereof include Disperbyk(R)-110, 111, 161, and 180 (all are trade names), BYK(R)-W996, BYK-W9010, BYK-W903, and BYK-W940 (all are trade names) manufactured by BYK Japan K.K. One of these wetting and dispersing agents can be used singly, or two or more thereof can be used in combination.

The content of the filler is preferably 0.0 parts by mass or more and 1600 parts by mass or less, more preferably 50 parts by mass or more and 1600 parts by mass or less, based on 100 parts by mass of the resin solid content in the resin composition. The content of the filler falls within the range described above, whereby the resulting resin composition tends to have better flame retardancy, low thermal expansibility, and toughness, etc.

### (Polymerization catalyst and curing accelerator)

The resin composition may be further supplemented with a polymerization catalyst, and/or a curing accelerator for appropriately adjusting a curing rate, in addition to the compounds and the resins described above.

Generally known compounds can be used as the polymerization catalyst and the curing accelerator. Specific examples thereof include: organic metal salts such as zinc octanoate, zinc naphthenate, cobalt naphthenate, copper naphthenate, acetylacetone iron, nickel octanoate, and manganese octanoate; phenolic compounds such as phenol, xylenol, cresol, resorcinol, catechol, octylphenol, and nonylphenol; alcohols such as 1-butanol and 2-ethylhexanol; imidazole derivatives such as 2-methylimidazole, 2-ethyl-4-methylimidazole, 2-phenylimidazole, 1-cyanoethyl-2-phenylimidazole, 1-cyanoethyl-2-ethyl-4-methylimidazole, 2-phenyl-4,5-dihydroxymethylimidazole, and 2-phenyl-4-methyl-5-hydroxymethylimidazole; derivatives of these imidazoles, such as adducts of carboxylic acids or acid anhydrides thereof; amine compounds such as dicyandiamide, benzyldimethylamine, and 4-methyl-N,N-dimethylbenzylamine; phosphorus compounds such as phosphine compounds, phosphine oxide compounds, phosphonium compounds, and diphosphine compounds; epoxy-imidazole adduct compounds; peroxides such as benzoyl peroxide, p-chlorobenzoyl peroxide, di-t-butyl peroxide, diisopropyl peroxycarbonate, and di-2-ethylhexyl peroxycarbonate; and azo compounds such as azobisisobutyronitrile. Commercially available products of these polymerization catalysts and curing accelerators may be used. Examples of such commercially available products include Amicure(R) PN-23 (trade name, manufactured by Ajinomoto Fine-Techno Co., Inc.), Novacure(R) HX-3721 (trade name, manufactured by Asahi Kasei Corp.), and Fujicure(R) FX-1000 (trade name, manufactured by Fuji Kasei Kogyo Co., Ltd.). One of these polymerization catalysts and curing accelerators can be used singly, or two or more thereof can be used in combination.

The contents of the polymerization catalyst and the curing accelerator can be appropriately adjusted in consideration of the degree of curing of resins, the viscosity of the resin composition, and the like and are each usually 0.005 parts by mass or more and 10 parts by mass or less based on 100 parts by mass of the resin solid content in the resin composition.

### (Other additives)

The resin composition may be optionally supplemented with an additive other than the compounds and the resins described above and the polymerization catalyst and the curing accelerator.

Examples of such an additive that may be contained therein include known additives including various polymer compounds such as thermosetting resins other than the compounds and the resins described above, thermoplastic resin, and their oligomers and elastomers, coloring pigments, antifoaming agents, surface adjusters, frame retardants, solvents, ultraviolet absorbents, antioxidants, photopolymerization initiators, fluorescent whitening agents, photosensitizing agents, dyes, pigments, thickeners, lubricants, flow modifiers, dispersing agents, leveling agents, gloss agents, and polymerization inhibitors. One of these additives can be used singly, or two or more thereof can be used in combination.

The content of each additive is usually 0.005 parts by mass or more and 10 parts by mass or less based on 100 parts by mass of the resin solid content in the resin composition.

### (Organic solvent)

The resin composition may optionally contain an organic solvent. In this case, the resin composition can be used as a form in which at least a portion, preferably the whole, of each resin component mentioned above is dissolved or compatibilized in an organic solvent (solution or varnish).

A generally known solvent can be used as the organic solvent as long as the solvent is capable of dissolving or compatibilizing at least a portion, preferably the whole, of each resin component mentioned above. Specific examples thereof include: ketone solvents such as acetone, methyl ethyl ketone, methyl isobutyl ketone, and cyclohexanone; cellosolve solvents such as propylene glycol monomethyl ether and propylene glycol monomethyl ether acetate; ester solvents such as methyl lactate, methyl acetate, ethyl acetate, butyl acetate, isoamyl acetate, ethyl lactate, methyl methoxypropionate, and methyl hydroxyisobutyrate; polar solvents such as amides including dimethylacetamide and dimethylformamide; alcohol solvents such as methanol, ethanol, isopropanol, and 1-ethoxy-2-propanol; and aromatic hydrocarbons such as toluene, xylene, and anisole. One of these organic solvents can be used singly, or two or more thereof can be used in combination.

The resin composition can be obtained by mixing the cyanate ester compound of the present embodiment and optionally other components together with an organic solvent using a known mixer, for example, a high-speed mixer, a Nauta mixer, a ribbon blender, a kneader, an intensive mixer, a universal mixer, a dissolver, or a static mixer. For mixing, a method for adding the cyanate ester compound, each additive, and the solvent is not particularly limited.

### [Purpose]

The cyanate ester compound and the resin composition of the present embodiment are suitably used in, for example, cured products, prepregs, laminates, metal foil-clad laminates, multilayer boards, materials for encapsulations, fiber-reinforced composite materials, adhesives, resin composite sheets, films, and printed wiring boards. The cyanate ester compound and the resin composition have, for example, low thermal expansibility, flame retardancy, and heat resistance and are therefore useful as highly functional polymer materials. The cyanate ester compound and the resin composition can be used as, for example, materials excellent in thermal, electric, and mechanical physical properties. Examples of such materials include electrical insulating materials, materials for encapsulations, adhesives, laminating materials, resists, and buildup laminate materials as well as fixation materials, structural members, reinforcing agents, templating materials, etc. in fields such as civil engineering and construction, electrical and electronics, automobiles, railroads, shipping, aircrafts, sport goods, and arts and crafts. Among them, the cyanate ester compound and the resin composition are suitable for electrical insulating materials, materials for semiconductor encapsulations, adhesives for electronic components, aircraft structural members, satellite structural members, and railroad vehicle structural members, which are required to have low thermal expansibility, flame retardance, and a high level of mechanical strength.

### [Cured product]

The cured product of the present embodiment is obtained by curing the resin composition of the present embodiment.

A method for producing the cured product can involve, for example, melting the resin composition or dissolving the resin composition in a solvent, and then injecting the resultant into a mold, followed by curing under usual conditions using heat, light, or the like. For heat curing, the curing temperature is preferably in the range of 120°C or higher and 300°C or lower from the viewpoint of allowing the curing to progress efficiently and preventing the deterioration of the resulting cured product. For photocuring, the curing is preferably performed in a light wavelength range of, for example, 100 nm or more and 500 nm or less in which the curing progresses efficiently with a photopolymerization initiator or the like.

### [Prepreg]

The prepreg has a base material and the resin composition of the present embodiment, the base material being impregnated or coated with the resin composition. The prepreg can be used as an insulation layer for printed wiring boards and a material for a semiconductor package.

### (Base material)

A generally known material can be appropriately selected and used as the base material, depending on performance required for the prepreg, for example, strength, a water absorption rate, and a coefficient of thermal expansion. Specific examples thereof include glass fiber base materials, synthetic fiber base materials, organic fiber base materials, and inorganic fiber base materials. Examples of the glass fiber constituting the glass fiber base material include A glass, C glass, D glass, E glass, H glass, L glass, NE glass, Q glass, S glass, T glass, UN glass, and spherical glass. Examples of the synthetic fiber constituting the synthetic fiber base material include: polyamide resin fibers such as polyamide resin fiber, aromatic polyamide resin fiber, and wholly aromatic polyamide resin fiber; polyester resin fibers such as polyester resin fiber, aromatic polyester resin fiber, and wholly aromatic polyester resin fiber; polyimide resin fiber; and fluororesin fiber. Examples of the organic fiber base material include paper base materials composed mainly of kraft paper, cotton linter paper, mixture paper of linter and kraft pulp, or the like. Examples of the inorganic fiber constituting the inorganic fiber base material include non-glass inorganic fibers such as quartz. Examples of the shape of the base material include woven fabrics, nonwoven fabrics, rovings, chopped strand mats, and surfacing mats. One of these base materials can be used singly, or two or more thereof can be used in combination. The thickness of the base material is preferably in the range of 0.01 mm or more and 0.2 mm or less for the purpose of laminates. When the thickness of the base material falls within the range described above, a woven fabric treated by opening or compaction is suitable from the viewpoint of dimensional stability. A glass woven fabric surface-treated with a silane coupling agent such as epoxysilane or aminosilane is further preferred from the viewpoint of moisture-absorbing heat resistance. Also, a liquid-crystal polyester woven fabric is preferred from the viewpoint of electrical properties.

A generally known method can be appropriately applied to a method for producing the prepreg. For example, resin varnish is prepared using the resin composition, and the prepreg can be produced by the application of a method of dipping the base material in the resin varnish, a method of coating the base material with the resin varnish using various coaters, a method of spraying the resin varnish to the base material using a spray, or the like. Among them, the method of dipping the base material in the resin varnish is preferred. The impregnating properties of the resin composition for the base material can thereby be improved. In the case of dipping the base material in the resin varnish, a usual impregnation or coating facility can be used. For example, a method can be applied thereto which involves impregnating or coating the inorganic and/or organic fiber base material with the resin varnish using an impregnation or coating facility, followed by drying at 120°C or higher and 220°C or lower for approximately 2 minutes or longer and 15 minutes or shorter to produce a prepreg through conversion into B stage. In this respect, the amount of the resin composition attached to the base material, i.e., the amount of the resin composition (including the filler) based on the total amount (100% by mass) of the prepreg after semi-curing, is preferably in the range of 20% by mass or more and 99% by mass or less.

### [Laminate]

The laminate has a layer containing at least one or more prepregs, and metal foil laminated on one side or both sides of the layer.

A generally known method can be appropriately applied to a method for producing the laminate. Such a production method can involve, for example, laminating the prepreg and the metal foil, followed by molding by heating and pressurization to obtain a laminate. In this respect, the heating temperature is preferably 65°C or higher and 300°C or lower, more preferably 120°C or higher and 270°C or lower. The pressurization pressure is preferably 2.0 MPa or higher and 5.0 MPa or lower, more preferably 2.5 MPa or higher and 4.0 MPa or lower. The laminate may be used as a metal foil-clad laminate and a multilayer board.

### [Metal foil-clad laminate]

The metal foil-clad laminate contains a layer of at least one or more prepregs laminated with metal foil disposed on one side or both sides thereof. Specifically, the metal foil-clad laminate can be prepared by layering one or more prepregs, and disposing metal (e.g., copper or aluminum) foil on one side or both sides thereof, followed by lamination. Examples of the metal foil include metal foil for use in materials for printed wiring boards. Such metal foil is preferably copper foil such as rolled copper foil or electrolytic copper foil. The thickness of the metal foil is preferably 2.0 µm or more and 70 µm or less, more preferably 3.0 µm or more and 35 µm or less. A usual approach for laminates and multilayer boards for printed wiring boards can be applied to molding conditions. The metal foil-clad laminate can be produced by using, for example, a multiplaten press, a multiplaten vacuum press, a continuous molding machine, or an autoclave molding machine and performing lamination at a temperature of 180°C or higher and 350°C or lower and a surface pressure of 20 kg/cm² or more and 100 kg/cm² or less for a heating time of 100 minutes or longer and 300 minutes or shorter.

### [Multilayer board]

The multilayer board is obtained by laminating the prepreg and a separately prepared wiring board for an inner layer in combination.

A method for producing the multilayer board can involve, for example, disposing 35µm copper foil on both sides of one prepreg, laminating the resultant under the conditions described above, then forming an inner layer circuit, carrying out the black oxide treatment of the circuit to form an inner layer circuit board, then alternately disposing this inner layer circuit board and the prepreg one by one, further disposing copper foil on the outermost layer, and laminating the resultant, preferably in vacuum, under the conditions described above to prepare a multilayer board.

### [Material for encapsulation]

The material for encapsulation contains the resin composition.

A generally known method can be appropriately applied to a method for producing the material for encapsulation. Examples of such a production method include a method of mixing the resin composition with various known additives that are generally used for the purpose of materials for encapsulations, or a solvent or the like using a known mixer. For mixing, a generally known method can be appropriately applied to a method for adding the cyanate ester compound, various additives, and the solvent.

### [Fiber-reinforced composite material]

The fiber-reinforced composite material contains the resin composition and a reinforcement fiber.

A generally known fiber can be used as the reinforcement fiber. Specific examples thereof include carbon fiber, glass fiber, aramide fiber, boron fiber, PBO fiber, high-strength polyethylene fiber, alumina fiber, and silicon carbide fiber. The form or arrangement of the reinforcement fiber is not particularly limited and can be appropriately selected from, for example, woven fabrics, unwoven fabrics, mats, knits, braided cords, unidirectional strands, rovings, and chopped strands. Alternatively, a preform (laminated woven ground fabrics made of the reinforcement fiber, or an integrally sutured product thereof using stitching yarn, or a fiber structure such as a three-dimensional woven fabric or a knitted or braided fabric) may be applied to the form of the reinforcement fiber.

A generally known method can be appropriately applied to a method for producing the fiber-reinforced composite material. Specific examples thereof include liquid composite molding methods, resin film infusion methods, filament winding methods, hand lay-up methods, and pultrusion methods. Among them, a resin transfer molding method, which is one of the liquid composite molding methods, is adaptable to various purposes because materials other than preforms, such as a metal plate, a foam core, and a honeycomb core can be loaded in molds in advance. Therefore, the resin transfer molding method is preferably used in the large-scale production of composite materials having a relatively complicated shape in a short time.

### [Adhesive]

The adhesive contains the resin composition of the present embodiment.

A generally known method can be appropriately applied to a method for producing the adhesive. Examples of such a production method include a method of mixing the resin composition with various known additives that are generally used for the purpose of adhesives, or a solvent or the like using a known mixer. For mixing, a generally known method can be appropriately applied to a method for adding the cyanate ester compound, various additives, and the solvent.

### [Resin composite sheet]

The resin composite sheet contains a support and a resin layer disposed on the surface of the support, the resin layer containing the resin composition of the present embodiment. The resin composite sheet may be obtained by coating a support with a solution of the resin composition of the present embodiment dissolved in a solvent, followed by drying.

Examples of the support include: organic film base materials such as polyethylene films, polypropylene films, polycarbonate films, polyethylene terephthalate films, ethylene-tetrafluoroethylene copolymer films, and mold release films containing these films surface-coated with a mold release agent, and polyimide films; and plate-like materials such as conductor foil (e.g., copper foil and aluminum foil), glass plates, SUS plates, and FRP. Examples of the coating method include a method of coating the support with the solution of the resin composition dissolved in a solvent using a bar coater, a die coater, a doctor blade, a baker applicator, or the like. After drying, the support may be removed from the laminated sheet by peeling or etching to prepare a monolayer sheet (resin sheet). The monolayer sheet (resin sheet) can also be obtained without the use of the support by supplying the solution of the resin composition dissolved in a solvent into a mold having a sheet-like cavity, followed by drying or the like to form a sheet shape.

In the preparation of the monolayer or laminated sheet, the drying conditions for removing the solvent preferably involve a temperature of 20°C or higher and 200°C or lower for 1 minute or longer and 90 minutes or shorter because a low temperature is likely to cause the solvent to remain in the resin composition while a high temperature allows the curing of the resin composition to progress. The thickness of the resin layer in the monolayer or laminated sheet can be adjusted by the concentration of the solution of the resin composition and a coating thickness, and is preferably 0.1 µm or more and 500 µm or less because a larger coating thickness is generally likely to cause the solvent to remain during drying.

### [Film]

The film is obtained by molding the resin composition into a sheet.

Such a film can be used as, for example, a film for buildup or a dry film solder resist. Examples of the method for producing the film include a method of using a peelable plastic film as a base material and coating the plastic film with a solution of the resin composition dissolved in a solvent, followed by drying. The solvent can be dried off by heating at a temperature of 20°C or higher and 150°C or lower for 1 minutes or longer and 90 minutes or shorter. The film may be used in an uncured state obtained by merely drying off the solvent from the resin composition, or may be used, if necessary, in a semi-cured (converted into B stage) state.

### [Printed wiring board]

The printed wiring board contains an insulation layer and a conductor layer formed on the surface of the insulation layer, the insulation layer containing the resin composition. Specifically, the insulation layer is preferably constituted by an insulation layer containing the resin composition of the present embodiment.

The metal foil-clad laminate can be suitably used as the printed wiring board. The printed wiring board can be produced in accordance with an ordinary method. Hereinafter, one example of the method for producing the printed wiring board will be shown. First, a metal foil-clad laminate such as copper clad laminate is prepared. Next, the surface of the metal foil-clad laminate is subjected to etching treatment for the formation of an inner layer circuit to prepare an inner layer substrate. The inner layer circuit surface of this inner layer substrate is surface-treated, if necessary, for enhancing adhesion strength. Subsequently, a required number of layers of the prepreg is put on the resulting inner layer circuit surface. Metal foil for an outer layer circuit is laterally laminated thereon, followed by integral molding by heating under increased pressure. In this way, a multilayer laminate is produced in which the insulating layer composed of the base material and a cured product of the resin composition is formed between the inner layer circuit and the metal foil for an outer layer circuit. Subsequently, this multilayer laminate is processed by hole drilling for a through-hole or a via hole. Then, the inside walls of these holes are coated with a metal plating film for the community between the inner layer circuit and the metal foil for an outer layer circuit. The metal foil for an outer layer circuit is further subjected to etching treatment for the formation of the outer layer circuit to produce the printed wiring board.

### Examples

Hereinafter, the present invention will be described in more detail with reference to Examples. However, the present invention is not particularly limited by the following Examples.

### [Calculation and measurement methods]

### (1) Content of compound having a triazine ring

The content (% by area) of a compound having a triazine ring in a cyanate ester compound obtained in each of Examples and Comparative Examples was measured as described below.

Specifically, 2.0 g of a 2-butanone solution having a content of 50% by mass of the cyanate ester compound was dissolved in 50 g of tetrahydrofuran (solvent) to obtain a solution. Analysis was conducted by high performance liquid chromatography (Hitachi High-Tech Corp., high performance liquid chromatograph LachromElite (trade name)) after injection of 2.0 µL of the solution. TSKgel ODS-120T manufactured by Tosoh Corp. (25 cm in length × 4.6 mm in inside diameter) was used as a column, acetonitrile/water (80/20 volume ratio) was used as a mobile phase, a flow rate was set to 1.0 mL/min., a detection wavelength was set to 274 nm, and a column temperature was set to 35°C. The content of the compound having a triazine ring in the cyanate ester compound was calculated from a peak area ratio of a peak observed at a retention time (RT) of 1.5 minutes.

### (2) Peak top temperature

The temperature at an exothermic peak (peak top temperature, °C) of the cyanate ester compound obtained in each of Examples and Comparative Examples was measured as described below.

First, for pretreatment, 5.0 g of a 2-butanone solution having 50% by mass of the cyanate ester compound was concentrated under reduced pressure and further concentrated into dryness at 70°C for 1 hour to obtain 2.7 g of the cyanate ester compound. The peak top temperature (unit: °C) was measured by the observation of the exothermic behavior of the obtained cyanate ester compound under measurement conditions involving a start temperature of 40°C, an end temperature of 380°C, and a temperature increase rate of 3.0°C/min using a differential scanning calorimeter (SII NanoTechnology Inc., DSC7020 (trade name)). A higher peak top temperature means that polymerization reaction is more easily controlled.

### [Example 1]

A 0.3 L glass flask with an openable lid equipped with a stirrer was provided. In this flask, nitrogen was circulated at 16.0 L/hr per L of the container, and 40 g (hydroxy group equivalent: 230 g/eq., amount based on a hydroxy group: 0.17 mol) of 1-naphthol aralkyl resin (SN495V, trade name, manufactured by NIPPON STEEL Chemical & Material Co., Ltd.) as a hydroxy-substituted aromatic compound and 26.4 g (0.26 mol, an amount of 1.5 mol based on 1 mol of a hydroxy group of the 1-naphthol aralkyl resin) of triethylamine as a basic compound 1 were dissolved in 240 g of dichloromethane as an organic solvent 1 with the solution temperature kept at 0°C to prepare a solution 1.

While a mixed liquid (solution 2) containing 16.0 g (0.26 mol, 1.50 mol based on 1 mol of a hydroxy group of the 1-naphthol aralkyl resin) of cyanogen chloride as cyanogen halide, 37.4 g of dichloromethane as an organic solvent 2, 26.4 g (0.26 mol, 1.50 mol based on 1 mol of a hydroxy group of the 1-naphthol aralkyl resin) of 36% hydrochloric acid as hydrogen halide, and 163.8 g of water was stirred, the solution 1 obtained above was poured to the mixed liquid over 12 minutes with the liquid temperature kept at -3 to -1°C to obtain a solution 2. Then, the solution 2 was further stirred for 5 minutes with the liquid temperature kept at -3 to -1°C. Then, a solution 3 of 7.0 g (0.07 mol, 0.40 mol based on 1 mol of a hydroxy group of the 1-naphthol aralkyl resin) of triethylamine as a basic compound 2 dissolved in 7.0 g of dichloromethane as an organic solvent 3 was poured to the solution 2 over 2 minutes with the liquid temperature kept at -3 to -1°C to obtain a solution 4. Then, the reaction was completed by the further stirring of the solution 4 for 30 minutes with the liquid temperature kept at -3 to -1°C to obtain a reaction liquid 1. The pH on the aqueous phase (upper phase) side in the reaction liquid 1 was measured with a pH meter (IQ Scientific Instruments, Inc., IQ150 (trade name)) and was consequently 0.9.

Then, the reaction liquid 1 was left standing, and an organic phase (dichloromethane phase) as a lower phase and an aqueous phase as an upper phase were accurately separated at their interface so as not to mix these phases into each other. In this respect, both the phases were transparent.

Cyanogen chloride and dichloromethane were distilled off at a bottom liquid temperature of 41°C (highest temperature) by the heating and distillation of the obtained dichloromethane phase. Then, fresh dichloromethane in an amount corresponding to the mass of the portion distilled off was further added to the substrate. Then, washing was performed five times with 200 g of water. As a result of measuring the electrical conductivity of waste water from the fifth wash with water using a portable EC METER (HI8733N, trade name, manufactured by Hanna Instruments JAPAN K.K.), the electrical conductivity was 10 µS/cm. From the value of the electrical conductivity, it was confirmed that an ionic compound was able to be sufficiently removed by the washing with water.

The dichloromethane phase after washing with water was concentrated under reduced pressure, and solvent replacement with a 2-butanone solution was performed five times to obtain 86.9 g of a 2-butanone solution having 50% by mass of the cyanate ester compound of interest (compound represented by the formula (17)).

The content of the compound having a triazine ring (compound having a structure represented by the formula (18)) in the obtained cyanate ester compound was 1.4% by area on HPLC area percentage, and the peak top temperature was 278°C. These results are shown in Table 1.

### [Example 2]

An openable reaction vessel having 9 m³ stainless steel equipped with a stirring blade was provided in which supply piping and a valve for inert gas circulation were installed in a portion of the vessel and a vent line (discharge piping) was installed on the exit side. In the vessel, nitrogen was circulated at 0.60 L/hr per L of the vessel for 2 hours to create a nitrogen atmosphere in the vessel. Then, in this vessel, nitrogen was circulated at 0.35 L/hr per L of the vessel, and 1140 kg (hydroxy group equivalent: 230 g/eq., amount based on a hydroxy group: 4.96 kmol) of 1-naphthol aralkyl resin (SN495V, trade name, manufactured by NIPPON STEEL Chemical & Material Co., Ltd.) as a hydroxy-substituted aromatic compound and 752.33 kg (7.44 kmol, an amount of 1.5 mol based on 1 mol of a hydroxy group of the 1-naphthol aralkyl resin) of triethylamine as a basic compound 1 were dissolved in 6270 kg of dichloromethane as an organic solvent 1 with the solution temperature kept at 0°C to prepare a solution 1.

While a mixed liquid (solution 2) containing 487.48 kg (7.93 kmol, 1.60 mol based on 1 mol of a hydroxy group of the 1-naphthol aralkyl resin) of cyanogen chloride as cyanogen halide, 1137.46 kg of dichloromethane as an organic solvent 2, 828.28 kg (8.178 kmol, 1.65 mol based on 1 mol of a hydroxy group of the 1-naphthol aralkyl resin) of 36% hydrochloric acid as hydrogen halide, and 5135.31 kg of water was stirred, the solution 1 obtained above was poured to the mixed liquid over 240 minutes with the liquid temperature kept at -3 to -1°C to obtain a solution 2. Then, the solution 2 was further stirred for 5 minutes with the liquid temperature kept at -3 to -1°C. Then, a solution 3 of 200.62 kg (1.98 mol, 0.40 mol based on 1 mol of a hydroxy group of the 1-naphthol aralkyl resin) of triethylamine as a basic compound 2 dissolved in 200.62 kg of dichloromethane as an organic solvent 3 was poured to the solution 2 over 60 minutes with the liquid temperature kept at -3 to - 1°C to obtain a solution 4. Then, the reaction was completed by the further stirring of the solution 4 for 30 minutes with the liquid temperature kept at -3 to -1°C to obtain a reaction liquid 1. The pH on the aqueous phase (upper phase) side in the reaction liquid 1 was measured with a pH meter (IQ Scientific Instruments, Inc., IQ150 (trade name)) and was consequently 0.9.

Then, the reaction liquid 1 was left standing, and an organic phase (dichloromethane phase) as a lower phase and an aqueous phase as an upper phase were accurately separated at their interface so as not to mix these phases into each other. In this respect, both the phases were transparent.

Cyanogen chloride and dichloromethane were distilled off at a bottom liquid temperature of 41°C (highest temperature) by the heating and distillation of the obtained dichloromethane phase. Then, fresh dichloromethane in an amount corresponding to the mass of the portion distilled off was further added to the substrate. Then, washing was performed five times with 3000 kg of water. As a result of measuring the electrical conductivity of waste water from the fifth wash with water using a portable EC METER (HI8733N, trade name, manufactured by Hanna Instruments JAPAN K.K.), the electrical conductivity was 10 µS/cm. From the value of the electrical conductivity, it was confirmed that an ionic compound was able to be sufficiently removed by the washing with water.

The dichloromethane phase after washing with water was concentrated under reduced pressure, and solvent replacement with a 2-butanone solution was performed five times to obtain 2477 kg of a 2-butanone solution having 50% by mass of the cyanate ester compound of interest (compound represented by the formula (17)).

The content of the compound having a triazine ring (compound having a structure represented by the formula (18)) in the obtained cyanate ester compound was 1.2% by area on HPLC area percentage, and the peak top temperature was 280°C. These results are shown in Table 1.

### [Comparative Example 1]

A 0.3 L glass flask equipped with a stirrer in an open system was provided. In this flask, 40 g (hydroxy group equivalent: 230 g/eq., amount based on a hydroxy group: 0.17 mol) of 1-naphthol aralkyl resin (SN495V, trade name, manufactured by NIPPON STEEL Chemical & Material Co., Ltd.) as a hydroxy-substituted aromatic compound and 26.4 g (0.26 mol, an amount of 1.5 mol based on 1 mol of a hydroxy group of the 1-naphthol aralkyl resin) of triethylamine as a basic compound 1 were dissolved in 240 g of dichloromethane as an organic solvent 1 with the solution temperature kept at 0°C to prepare a solution 1.

While a mixed liquid (solution 2) containing 16.0 g (0.26 mol, 1.50 mol based on 1 mol of a hydroxy group of the 1-naphthol aralkyl resin) of cyanogen chloride as cyanogen halide, 37.4 g of dichloromethane as an organic solvent 2, 26.4 g (0.26 mol, 1.50 mol based on 1 mol of a hydroxy group of the 1-naphthol aralkyl resin) of 36% hydrochloric acid as hydrogen halide, and 163.8 g of water was stirred, the solution 1 obtained above was poured to the mixed liquid over 14 minutes with the liquid temperature kept at -3 to -1°C to obtain a solution 2. Then, the solution 2 was further stirred for 5 minutes with the liquid temperature kept at -3 to -1°C. Then, a solution 3 of 8.8 g (0.09 mol, 0.50 mol based on 1 mol of a hydroxy group of the 1-naphthol aralkyl resin) of triethylamine as a basic compound 2 dissolved in 8.8 g of dichloromethane as an organic solvent 3 was poured to the solution 2 over 3 minutes with the liquid temperature kept at -3 to -1°C to obtain a solution 4. Then, the reaction was completed by the further stirring of the solution 4 for 30 minutes with the liquid temperature kept at -3 to -1°C to obtain a reaction liquid 1. The pH on the aqueous phase (upper phase) side in the reaction liquid 1 was measured with a pH meter (IQ Scientific Instruments, Inc., 10150 (trade name)) and was consequently 0.3.

Then, the reaction liquid 1 was left standing, and an organic phase (dichloromethane phase) as a lower phase and an aqueous phase as an upper phase were accurately separated at their interface so as not to mix these phases into each other. In this respect, the aqueous phase was slightly turbid.

Cyanogen chloride and dichloromethane were distilled off at a bottom liquid temperature of 41°C (highest temperature) by the heating and distillation of the obtained dichloromethane phase. Then, fresh dichloromethane in an amount corresponding to the mass of the portion distilled off was further added to the substrate. Then, washing was performed five times with 200 g of water. As a result of measuring the electrical conductivity of waste water from the fifth wash with water using a portable EC METER (HI8733N, trade name, manufactured by Hanna Instruments JAPAN K.K.), the electrical conductivity was 10 µS/cm. the electrical conductivity was 10 µS/cm. From the value of the electrical conductivity, it was confirmed that an ionic compound was able to be sufficiently removed by the washing with water.

The dichloromethane phase after washing with water was concentrated under reduced pressure, and solvent replacement with a 2-butanone solution was performed five times to obtain 86.0 g of a 2-butanone solution having 50% by mass of the cyanate ester compound of interest (compound represented by the formula (17)).

The content of the compound having a triazine ring (compound having a structure represented by the formula (18)) in the obtained cyanate ester compound was 4.8% by area on HPLC area percentage, and the peak top temperature was 265°C. These results are shown in Table 1.

### [Comparative Example 2]

A 0.3 L glass flask with an openable lid equipped with a stirrer was provided. In this flask, air was circulated at 1.0 L/hr per L of the container, and 40 g (hydroxy group equivalent: 230 g/eq., amount based on a hydroxy group: 0.17 mol) of 1-naphthol aralkyl resin (SN495V, trade name, manufactured by NIPPON STEEL Chemical & Material Co., Ltd.) as a hydroxy-substituted aromatic compound and 26.4 g (0.26 mol, an amount of 1.5 mol based on 1 mol of a hydroxy group of the 1-naphthol aralkyl resin) of triethylamine as a basic compound 1 were dissolved in 240 g of dichloromethane as an organic solvent 1 with the solution temperature kept at 0°C to prepare a solution 1.

While a mixed liquid (solution 2) containing 16.0 g (0.26 mol, 1.50 mol based on 1 mol of a hydroxy group of the 1-naphthol aralkyl resin) of cyanogen chloride as cyanogen halide, 37.4 g of dichloromethane as an organic solvent 2, 26.4 g (0.26 mol, 1.50 mol based on 1 mol of a hydroxy group of the 1-naphthol aralkyl resin) of 36% hydrochloric acid as hydrogen halide, and 163.8 g of water was stirred, the solution 1 obtained above was poured to the mixed liquid over 14 minutes with the liquid temperature kept at -3 to -1°C to obtain a solution 2. Then, the solution 2 was further stirred for 5 minutes with the liquid temperature kept at -3 to -1°C. Then, a solution 3 of 10.6 g (0.10 mol, 0.60 mol based on 1 mol of a hydroxy group of the 1-naphthol aralkyl resin) of triethylamine as a basic compound 2 dissolved in 10.6 g of dichloromethane as an organic solvent 3 was poured to the solution 2 over 4 minutes with the liquid temperature kept at -3 to -1°C to obtain a solution 4. Then, the reaction was completed by the further stirring of the solution 4 for 30 minutes with the liquid temperature kept at -3 to -1°C to obtain a reaction liquid 1. The pH on the aqueous phase (upper phase) side in the reaction liquid 1 was measured with a pH meter (IQ Scientific Instruments, Inc., IQ150 (trade name)) and was consequently 0.3.

Then, the reaction liquid 1 was left standing, and an organic phase (dichloromethane phase) as a lower phase and an aqueous phase as an upper phase were accurately separated at their interface so as not to mix these phases into each other. In this respect, the aqueous phase was slightly turbid.

Cyanogen chloride and dichloromethane were distilled off at a bottom liquid temperature of 41°C (highest temperature) by the heating and distillation of the obtained dichloromethane phase. Then, fresh dichloromethane in an amount corresponding to the mass of the portion distilled off was further added to the substrate. Then, washing was performed five times with 200 g of water. As a result of measuring the electrical conductivity of waste water from the fifth wash with water using a portable EC METER (HI8733N, trade name, manufactured by Hanna Instruments JAPAN K.K.), the electrical conductivity was 10 µS/cm. From the value of the electrical conductivity, it was confirmed that an ionic compound was able to be sufficiently removed by the washing with water.

The dichloromethane phase after washing with water was concentrated under reduced pressure, and solvent replacement with a 2-butanone solution was performed five times to obtain 85.1 g of a 2-butanone solution having 50% by mass of the cyanate ester compound of interest (compound represented by the formula (17)).

The content of the compound having a triazine ring (compound having a structure represented by the formula (18)) in the obtained cyanate ester compound was 5.0% by area on HPLC area percentage, and the peak top temperature was 263°C. These results are shown in Table 1.

**[Table 1]**

| | Starting material solution preparation step | | Cyanate ester compound | |
|---|---|---|---|---|
| | Atmosphere in container | Gas flow rate per L of container | Content of compound having triazine ring | Peak top temperature |
| | - | (L/hr) | (% by area) | (°C) |
| Example 1 | Nitrogen | 16.0 | 1.4 | 278 |
| Example 2 | Nitrogen | 0.35 | 1.2 | 280 |
| Comparative Example 1 | (Open) | | 4.8 | 265 |
| Comparative Example 2 | Air | 1.0 | 5.0 | 263 |

The present application claims the priority based on the Japanese patent application filed in the Japan Patent Office on July 27, 2023 (Japanese Patent Application No. 2023-122171), the contents of which are incorporated herein by reference.

### Industrial Applicability

The cyanate ester compound of the present invention is suitably used in, for example, cured products, prepregs, laminates, metal foil-clad laminates, multilayer boards, materials for encapsulations, fiber-reinforced composite materials, adhesives, resin composite sheets, films, and printed wiring boards.

## Claims

1. A cyanate ester compound having two or more cyanate groups in a molecule, wherein a content of a compound having a triazine ring is 4.0% by area or less on HPLC area percentage.

2. The cyanate ester compound according to claim 1, wherein the cyanate ester compound is a compound represented by the following formula (1) or a compound represented by the following formula (2): 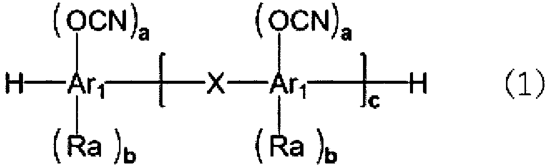 wherein
each Ar₁ independently represents an aromatic ring,
each Ra independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms,
a represents the number of cyanate groups bonded to Ar₁ and each independently represents an integer of 1 or larger and 3 or smaller,
b represents the number of Ra bonded to Ar₁ and each independently represents a number determined by subtracting (a + 2) from the number of substitutable substituents in Ar₁,
c is an integer of 1 or larger and 50 or smaller, and
each X independently represents a single bond, a divalent organic group having 1 or more and 50 or less carbon atoms in which a hydrogen atom is optionally replaced with a heteroatom, a divalent organic group having 1 or more and 10 or less nitrogen atoms, a carbonyl group, a carboxy group, a carbonyl dioxide group, a sulfonyl group, a divalent sulfur atom, or a divalent oxygen atom, and 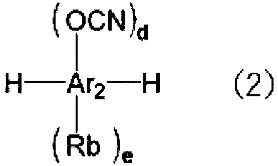 wherein
Ar₂ represents an aromatic ring,
each Rb independently represents a hydrogen atom, an alkyl group having 1 or more and 6 or less carbon atoms, an alkenyl group having 2 or more and 6 or less carbon atoms, an aryl group having 6 or more and 12 or less carbon atoms, or an alkoxy group having 1 or more and 4 or less carbon atoms,
d represents the number of cyanate groups bonded to Ar₂ and is an integer of 2 or larger and 3 or smaller, and
e represents the number of Rb bonded to Ar₂ and represents a number determined by subtracting (d + 2) from the number of substitutable substituents in Ar₂.

3. The cyanate ester compound according to claim 2, wherein each X in the formula (1) is independently selected from the group consisting of
a divalent organic group having 1 or more and 50 or less carbon atoms, the divalent organic group being represented by the following formula (3): 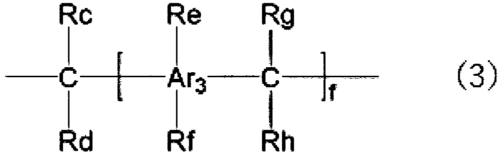 wherein each Ar₃ independently represents an aromatic ring, Rc, Rd, Rg, and Rh each independently represent a hydrogen atom, an alkyl group having 1 or more and 6 or less carbon atoms, or an aryl group having 6 or more and 12 or less carbon atoms, Re and Rf each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and f represents an integer of 0 or larger and 5 or smaller,
a divalent organic group having 1 or more and 50 or less carbon atoms, the divalent organic group being represented by the following formula (4): 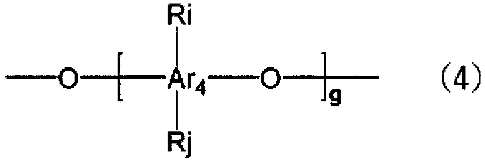 wherein each Ar₄ independently represents an aromatic ring, Ri and Rj each independently represent a hydrogen atom, an alkyl group having 1 or more and 6 or less carbon atoms, an aryl group having 6 or more and 12 or less carbon atoms, or an alkoxy group having 1 or more and 4 or less carbon atoms, and g represents an integer of 0 or larger and 5 or smaller, and
divalent groups represented by the following formulas (5) to (14): 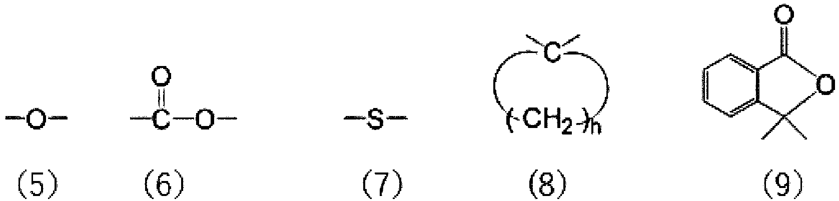 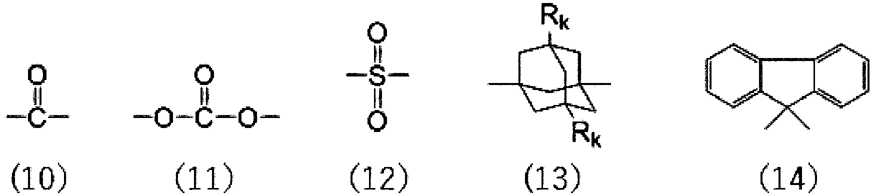 wherein h in the formula (8) represents an integer of 4 or larger and 7 or smaller, and each Rk in the formula (13) independently represents a hydrogen atom or an alkyl group having 1 or more and 6 or less carbon atoms.

4. The cyanate ester compound according to claim 1, wherein the cyanate ester compound is a compound represented by the following formula (15) or a compound represented by the following formula (16): 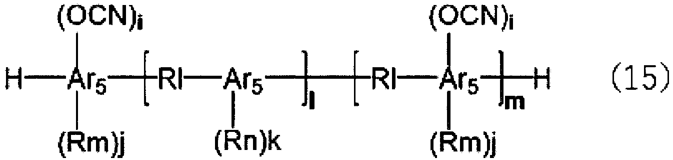 wherein
each Ar₅ independently represents an aromatic ring,
each RI independently represents a methylene group, a methyleneoxy group, a methyleneoxymethylene group, or an oxymethylene group, or two or more of these groups linked to each other,
Rm and Rn each independently represent a hydrogen atom, an alkyl group having 1 or more and 6 or less carbon atoms, an aryl group having 6 or more and 12 or less carbon atoms, or an alkoxy group having 1 or more and 4 or less carbon atoms,
i represents the number of cyanate groups bonded to Ar₅ and each independently represents an integer of 1 or larger and 3 or smaller,
j represents the number of Rm bonded to Ar₅ and represents a number determined by subtracting (i + 2) from the number of substitutable substituents in Ar₅,
k represents the number of Rn bonded to Ar₅ and represents a number determined by subtracting 2 from the number of substitutable substituents in Ar₅,
I represents an integer of 1 or larger, m represents an integer of 1 or larger, and each repeating unit is arbitrarily arranged, and 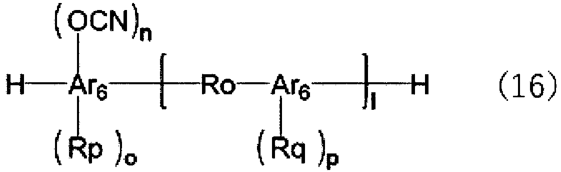 wherein
each Ar₆ independently represents an aromatic ring,
each Ro independently represents a methylene group, a methyleneoxy group, a methyleneoxymethylene group, or an oxymethylene group, or two or more of these groups linked to each other,
Rp and Rq each independently represent a hydrogen atom, an alkyl group having 1 or more and 6 or less carbon atoms, an aryl group having 6 or more and 12 or less carbon atoms, or an alkoxy group having 1 or more and 4 or less carbon atoms,
n represents the number of cyanate groups bonded to Ar₆ and is an integer of 2 or larger and 3 or smaller,
o represents the number of Rp bonded to Ar₆ and represents a number determined by subtracting (n + 2) from the number of substitutable substituents in Ar₆,
p represents the number of Rq bonded to Ar₆ and represents a number determined by subtracting 2 from the number of substitutable substituents in Ar₆, and
i represents an integer of 1 or larger.

5. The cyanate ester compound according to claim 1, wherein the cyanate ester compound is a compound represented by the following formula (17): 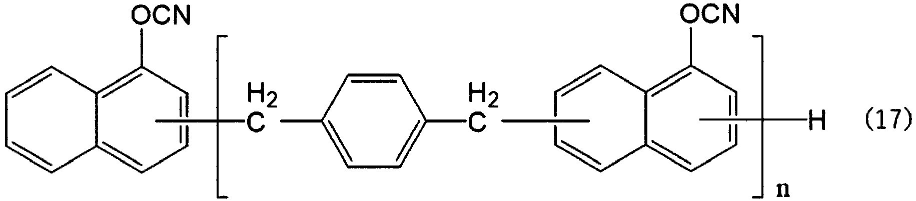 wherein n represents an integer of 1 or larger and 50 or smaller.

6. The cyanate ester compound according to claim 1, wherein the compound having a triazine ring is a compound having a structure represented by the following formula (18): 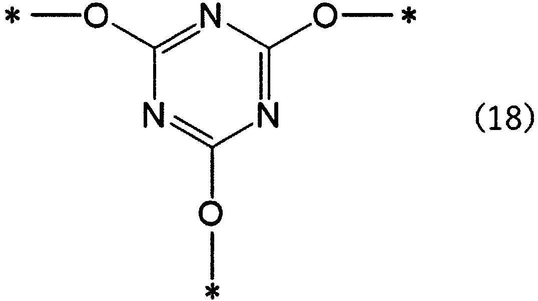 wherein * represents a binding site.

7. A method for producing a cyanate ester compound according to claim 1, comprising:
a starting material solution preparation step of preparing a first solution containing a hydroxy-substituted aromatic compound, a basic compound, and an organic solvent in a container, wherein the first solution is prepared while an inert gas is circulated at 0.1 L/hr or more and 20 L/hr or less per L of the container; and
a cyanation step of bringing the first solution into contact with a second solution containing cyanogen halide, hydrogen halide, an organic solvent, and water, and performing cyanation through reaction to obtain a reaction liquid containing the cyanate ester compound.

8. The method for producing a cyanate ester compound according to claim 7, wherein in the starting material solution preparation step, a temperature of the first solution is 5.0°C or lower.

9. The method for producing a cyanate ester compound according to claim 7, wherein
the cyanation step comprises
bringing the first solution into contact with the second solution to obtain a reaction liquid, and then pouring a third solution containing a basic compound and an organic solvent to the reaction liquid.

10. The method for producing a cyanate ester compound according to claim 7, wherein
in the cyanation step,
an amount of a starting material charged for the cyanogen halide is 0.5 mol or more and 5.0 mol or less based on 1 mol of a hydroxy group of the hydroxy-substituted aromatic compound.

11. The method for producing a cyanate ester compound according to claim 7, wherein in the cyanation step, a temperature of the reaction liquid is 5.0°C or lower.

12. A resin composition comprising the cyanate ester compound according to claim 1.

13. A cured product obtained by curing the resin composition according to claim 12.
